# EUROPEAN PATENT SPECIFICATION

(11) **EP 2 105 433 B1**
(45) Date of publication and mention of the grant of the patent: **22.09.2010**
(21) Application number: 08152925.7
(22) Date of filing: 18.03.2008
(51) Int. Cl.: C07C 237/22, C07D 209/20, C07D 403/14, A61K 31/16

(54) **Modular scaffold for the design of specific molecules for the use as peptidomimetics and inhibitors of protein interaction**
Modulares Gerüst zur Gestaltung von spezifischen Molekülen zur Verwendung als Pepitmimetika und Hemmer für die Proteininteraktion
Échafaudage modulaire pour la conception de molécules spécifiques pour l'utilisation de peptidomimétiques et d'inhibiteurs de l'interaction protéique

(43) Date of publication of application: 30.09.2009
(73) Proprietor: Technische Universität Dresden, 01069 Dresden (DE)
(72) Inventor: Baldauf, Carsten, 01099, Dresden (DE); Pisabarro, Maria Teresa, 01307, Dresden (ES)
(74) Representative: Kailuweit, Frank

(56) References cited:
- CHUNG Y J ET AL: "A beta-peptide reverse turn that promotes hairpin formation" JOURNAL OF THE AMERICAN CHEMICAL SOCIETY, AMERICAN CHEMICAL SOCIETY, WASHINGTON, DC.; US, US, vol. 120, no. 40, 1 January 1998 (1998-01-01), pages 10555-10556, XP002957220 ISSN: 0002-7863
- JOSEPH M. LANGENHAN ET AL.: "Parallel Sheet Secondary Structure in beta-Peptides" ANGEWANDTE CHEMIE. INTERNATIONAL EDITION., vol. 115, 2003, pages 2504-2507, XP002490460 DEVERLAG CHEMIE. WEINHEIM.
- C. BALDAUF ET AL.: "Stable Hairpins with beta-Peptides: Route to Tackle Protein-Protein Interactions" JOURNAL OF PHYSICAL CHEMISTRY B, AMERICAN CHEMICAL SOCIETY, vol. 112, 30 May 2008 (2008-05-30), pages 7581-7591, XP002490461 WASHINGTON , DC; US

## Description

### Field of the invention:

This invention relates to synthetic compounds comprising oligomers of β-amino acids with well defined secondary structural preferences. The compounds according to the invention are useful for .biotechnology and pharmacy, in particular as recognition blocks or molecular receptors that mimic protein recognition features and that are useful to modulate protein interactions.

### State of the art:

The modular nature of proteins and the intrinsic elasticity of their domains are essential features of biological function regulation, as they are often translated into conformational properties associated to molecular recognition (critically affecting their ability to bind other molecules; i.e. affinity/specificity) and variation of binding modes (critically affecting the way they bind to other molecules). Thus, molecules able to target molecular recognition and influence biological function may represent powerful therapeutic agents. However, development of small molecules as modulators of protein interactions is not a straightforward process due to the fact that protein interfaces found in signaling pathways are flat and often relatively large surface, which exceeds the potential binding area covered by a small molecule (Zeng, J. Comb Chem High Throughput Screen 2000, 3, 355.). Furthermore, in signaling interactions the same surface may bind to structurally diverse targets. This renders it difficult to find a molecule that can specifically modulate a path without affecting others, raising the importance of defining determinants of conformation/affinity/specificity in order modulate biological function. Protein recognition may be concentrated in a few key residues (*hot spots*) arranged in a particular three-dimensional (3D) manner. This fact has lead to the concept of "protein-surface mimetics", compounds retaining those essential functionalities and the ability to display them in a 3D pattern complementary to the molecules they recognize (Fletcher, S.; Hamilton, A. D. Curr Opin Chem Biol 2005, 9, 632).

However, peptides are usually bad drug candidates due to three main reasons: 1) intrinsic conformational flexibility in solution, i.e. the minimum length that peptides need to adopt a reasonably stable conformation is rather high, 2) poor stability against proteases, and 3) limited functionality due to the limited chemical diversity of the 20 canonical amino acid side chains. Therefore, there is a need for molecules exhibiting better *drug-like* properties than peptides in order to specifically target protein conformation and influence biological function. α-Helices, β-hairpins and polyproline-II helices (PPII) are the 3D structures most commonly used in cellular signaling protein recognition, accomplishing binding specificity by sequence diversity. β-Hairpins are secondary structural motifs often found in protein recognition and, therefore, of great pharmacological interest. The need to mimic these common 3D templates and the aforementioned shortcomings of peptides/mini-protein scaffolds for pharmacological purposes brings the focus on oligomers consisting of non-natural building blocks (Gellman, S. H. Accounts in Chemical Research 1998, 31, 173; Hill, D. J. et al. Chemical Reviews 2001, 101, 3893), so called foldamers. One possible way to derive such compounds is the homologation of the α-amino acid backbone to β-, γ-, and δ-amino acids. Oligomers of such non-natural amino acids are accordingly called β-, γ- and δ-peptides. β-Peptides consist of β-amino acids, where the amino group is bound to the β-carboy rather than to the α-carbon as in the 20 standard biological amino acids.

Because the backbones of β-peptides are longer than those of peptides that consist of α-amino acids, β-peptide form different secondary structures. Nevertheless, stabilization of conformations by H-bonds is of importance in this class of peptides, too. Substitution on the two C-atoms of the β-peptide backbone also has an effect on molecule conformation. In disubstituted β-amino acids the alkyl substituents at both the α and β positions in a β amino acid normally favor a gauche conformation about the bond between the α-carbon and β-carbon.

β-Peptid secondary structures can exhibit similarities to those of α-peptides or show completely new structural features. (Möhle, K. et al. Biopolymers 1999, 50, 167. Günther, R.; Hofmann, H.-J. Helv. Chim. Acta 2002, 85, 2149., Baldauf, C. et al. Angew. Chem. Int. Edit. 2004, 43, 1594. Dado, G. P.; Gellman, S. H. J. Am, Chem. Soc. 1994, 116, 1054. Appella, D. H. et al. J. Am. Chem. Soc. 1996, 118, 13.071. Seebach, D. et al. Helv. Chim. Acta 1996, 79, 913. Seebach, D.; et al.. Helv. Chim. Acta 1996, 79, 2043*.* Günther, R. et al. J. Phys. Chem. B 2001, 105, 5559.)

Many types of structures formed by β-peptides, such as 14-helix, 12-helix, 10/12-helix, 10-helix, C6-ribbon, and pleated-sheets have been reported. For instance, β-peptide consisting of homochiral β^{2,3}-amino acids (i.e. β-amino acids disubstituted on the C_{α} and C_{β} atom) have been shown to form 3₁₄-helical structures (Seebach et al. Helv. Chim Acta 1998, 81, 932-982), while heterochiral β^{2,3}-amino acids have been predicted to adopt extended conformations, and thus should favor the formation of pleated sheets (Seebach et al. Helv. Chim. Acta 1996, 79, 913-941). First attempts to obtain a β-peptide which forms a β-hairpin-like structure in methanol solution were made by Seebach and co-workers. However, this peptide does not fold into a stable hairpin-like structure as the two strands fail to converge enough to come into hydrogen bonding distance (Daura, X. et al J. Am. Chem. Soc. 2001, 123, 2393 - -s.S.2396.

The objective of the present invention is to provide synthetic compounds, which on one hand imitate the backbone structure of naturally occurring β-hairpin and, on the other hand, have improved characteristics, to be able to modulate functional interactions in biological systems.

### Summary of the invention:

To solve this objective the present invention provides a compound of the formula 1

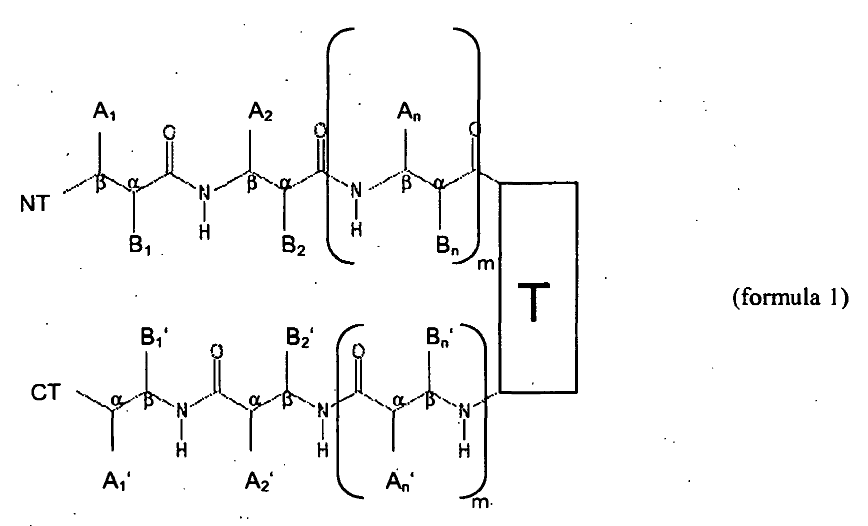

As shown in formula 1 the compound according to the invention comprises an N-terminal oligomer and a C-terminal oligomer, which are connected covalently to a turn motive T. Each of the two oligomers contains at least three β-amino acid building blocks (residues) as monomers. 'm + 2' represents the number of monomers in each oligomer and m is an integer from 1 to 6, preferably chosen from 1, 2, 3 and 4.

n and n' are the indexes of the third and further monomers in each oligomer. n and n' are integers from 3 to 8, preferably chosen from 3, 4, 5 and 6. In the N-terminal oligomer the building blocks are further referred to as residue 1 (with side chains A₁ and B₁), residue 2 (with side chains A₂ and B₂), and residue n for the third and subsequent building blocks with (with side chains Aₙ and Bₙ). Accordingly the residues in the C-terminal oligomer are named residue 1' (with side chains A₁' and B₁'), residue 2' (with side chains A₂' and B₂') and residue n' with (with side chains Aₙ' and Bₙ'). It should be clear that in case m > 1 the third and subsequent monomers in each oligomer (with different indexes n and n') may differ (bearing different side chains Aₙ, Bₙ, Aₙ' and Bₙ').

NT is the N-terminus of residue 1, which is either a free or a modified N-terminal amino group. CT is the C terminus of residue 1', which is either a free or a modified C-terminal carboxyl group.

The N-terminal oligomer and the C-terminal oligomer mainly consist of heterochiral β^{2,3}-amino acid residues (sometimes also referred to as *unlike* β-amino acid residues), meaning amino acid building blocks which are substituted on both the alpha (C_{α}) and the beta-carbon (C_{β}) atom, whereas the C_{α} and C_{β} are either in R,S or S,R conformation. According to the invention, almost all (meaning all or a maximum of m - 1) residues in each of the two oligomers are heterochiral β^{2,3}-amino acid building blocks, which are either uniformly in R,S or S,R conformation.

Consequently, each of the two oligomers contain at least 3 heterochiral β^{2,3}-amino acid residues. The side chains on the C_{α}- and the C_{β}-atom can in principle be chosen from side chains occurring in natural α-amino acids or be artificial side chains, preferably chosen from aliphatic or aromatic side chains with 1 to 10 C-atoms, which might be further substituted or not.

However, it is important that almost all of the side chains A₁, A₂ and Aₙ, B₁, B₂ and Bₙ, A₁, A₂. and Aₙ, B₁', B₂' and Bₙ' have more than 1 non-hydrogen atom. Therefore, according to the invention the side chains in all or at least m - 1 residues in each of the two oligomers have at least 2 non-hydrogen atoms, preferably chosen from carbon, nitrogen, oxygen, phosphor, selenium, sulphur and halogen atoms. Preferably, at least the non-hydrogen atom connected to the β-peptide backbone is a carbon.

Consequently in each oligomer the side chains of at least 3 heterochiral β^{2,3}-amino acid residues have at least 2 non-hydrogen atoms. In case m > 1 one of the side chains in one or two of the residues in each oligomer can be a hydrogen or a side chain with only one non-hydrogen atom, preferably chosen from carbon, nitrogen, oxygen, phosphor, selenium and sulphur. Thus, in case m = 2 one of the residues in each oligomer can be a β-amino acid residue, which is mono-substituted either on the C_{α}(a β²-amino acid residue) or the C_{β} (a β³-amino acid residue). In case m = 3 two of the side chains in one or two of the residues in each oligomer can be hydrogen or a side chain with only one non-hydrogen atoms, preferably chosen from carbon, nitrogen, oxygen, phosphor, selenium and sulphur. Thus, in case m = 3 two of the residues in each oligomer can be a β-amino acid residue, which is mono-substituted either on the C_{α} (a β²-amino acid residue) or the C_{β} (a β³-amino acid residue) and so on for m = 4 and higher.

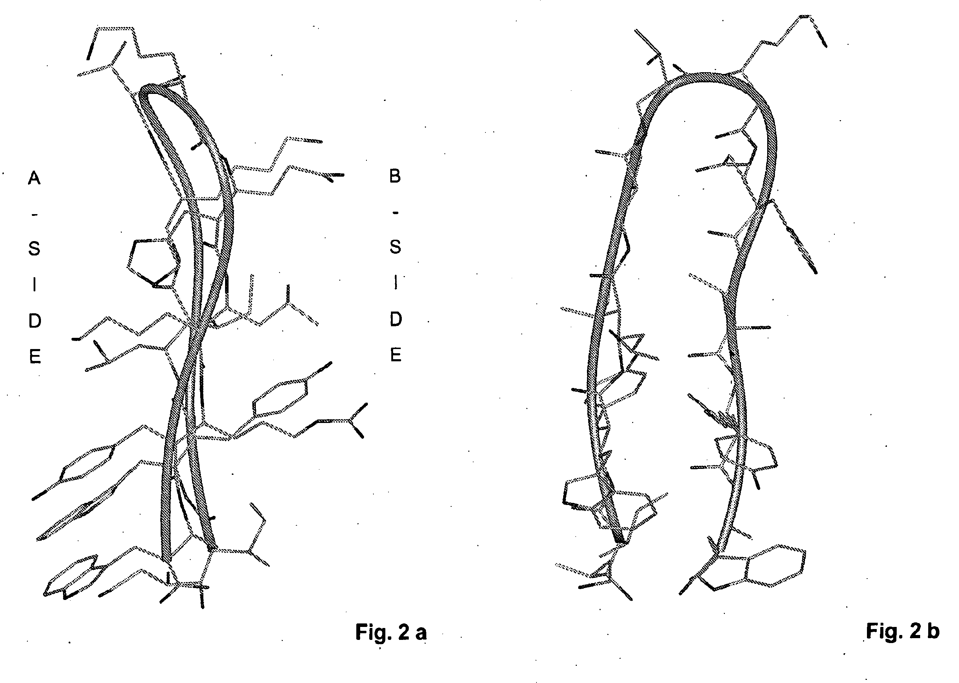

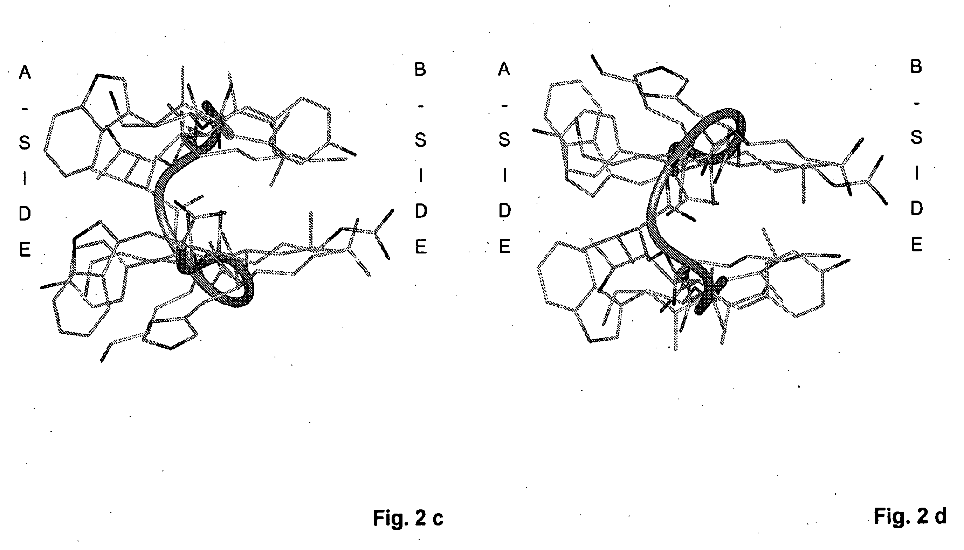

The compound of the invention folds advantageously into a β-hairpin like structure with two distinct sides (or faces) wherein the residues A₁, A₂ and Aₙ as well as the residues A₁', A₂ and Aₙ point to the one side of the β-hairpin (called A-side) and the residues B₁, 8₂ and Bₙ as well as the residues B₁^{'}, B₂^{'} and Bₙ^{'} point to the opposite side of the β-hairpin (called B-side) - see Fig. 2 and corresponding text.

At least four of the side chains chosen either from A₁ and A₁^{'}, A₂ and A₂^{'}, Aₙ and Aₙ^{'} or from B₁ and B₁^{'}, B₂ and B₂^{'}, Bₙ and Bₙ^{'}, form two pairs, wherein each non-prime member of the pair interacts with its prime () counterpart. These interactions are due to intramolecular forces or more precisely interstrand forces (as the oligomers are covalently linked by the turn motive T) preferably non-covalent forces.

It should be noted that A₁, A₂ Aₙ are substitutions on the C_{α} atom, and the corresponding side chains A₁^{'}, A₂^{'}, Aₙ^{'} are substitutions on the C_{β} atom. B₁, B₂ Bₙ are substitutions on the C_{β} atom, and the corresponding side chains B₁^{'}, B₂^{'}, Bₙ^{'} are substitutions on the C_{α} atom.

These interacting pairs advantageously stabilize the β-hairpin like structure in a kind of molecular hook-and-loop fastener ("molecular velcro"), folding like a molecular zipper. However, unlike those fasteners, which are usually formed out of a great number of uniform pairs of hooks and loops, at least two of the pairs in the compounds of the invention are not uniform, as they interact via different chemical mechanisms.

At least one pair forms a polar interaction, including electrostatic forces or dipole-dipole interactions, hydrogen bonds, electrostatic interactions (including coulomb and ionic interactions, like salt-bridges), electron donor-acceptor complexes and Charge-transfer-complexes, like a Cation-π-interaction, or a disulphide bridge.

At least a second pair interacts via nonpolar forces, preferable via van der Waals forces (also referred to as London Dispersion Forces), π-π-interactions or other hydrophobic interactions.

Advantageously the two interacting pairs lead to a fast formation (spontaneous folding) and stabilization of the β-hairpin structure.

Preferably the compound contains a third interacting pair, which forms preferably a polar interaction or a chemical bond or alternatively interacts via nonpolar forces, as described above.

Preferably the residues 1 and 1' and 3 and 3' contain (either on the A or B side) side chains interacting via polar forces. The residue 2 and 2' preferably contain (either on the A or B side) side chains interacting via non polar forces.

The major differences between β-hairpins in native proteins (comprising oligomers of alpha-amino acids) and the compounds according to the invention, further referred to as β-peptide hairpins (as they comprise oligomers of β-amino acids) are:
i) β-hairpins are slightly twisted around their longitudinal axis, the β-peptide hairpins according to the invention are not;
ii) the β-peptide hairpins according to the invention form all backbone H-bonds in the same direction relative to the sequence;
iii) each residue in the β-peptide hairpins according to the invention presents one side chain to each face of the hairpin, whereas the side chains of the residues in β-hairpins point alternating to one or the other side;
iv) β-peptide hairpins are not susceptible to cleavage by proteases.

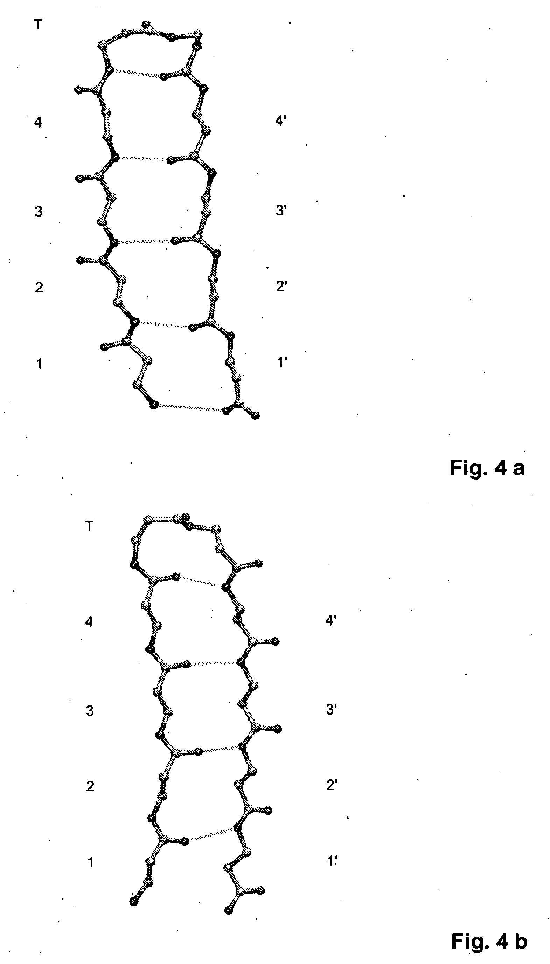

Advantageously, the formation of β-peptide hairpins according to the invention by spontaneous folding is faster and more stable in aqueous solution than the folding of naturally occurring β-hairpins. In contrast to β-hairpins consisting out of α-amino acids (like the comparative examples G4 and Trpzip2) the compounds according to the invention have no alternative folds to hairpin-like conformations. (refer to example 2 and Fig. 4 to 6).

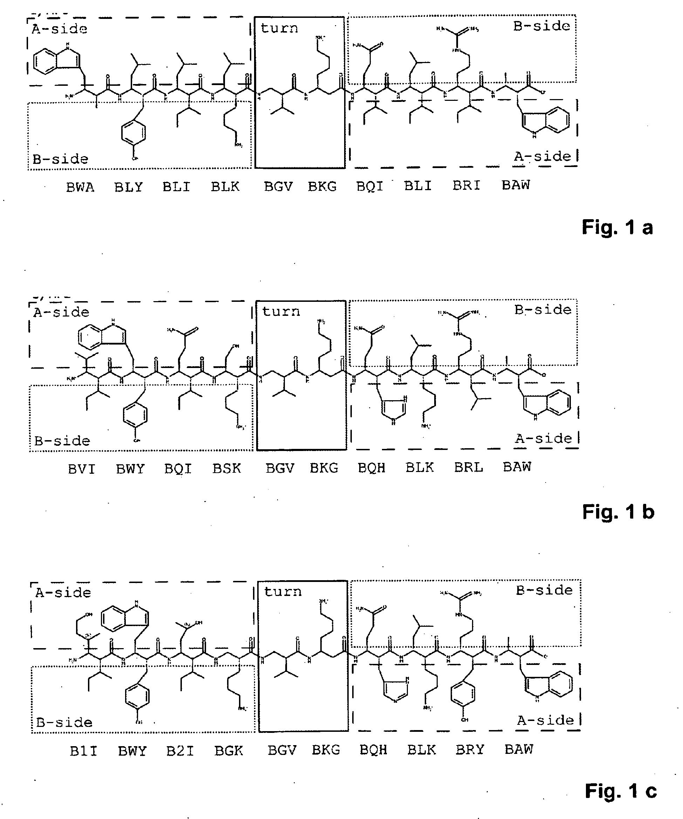

Each residue in the β-peptide hairpins according to the invention presents one side chain to every face of the hairpin (see also Fig. 1a to 1c and Fig. 2a to 2d).

The side chains A₁, A₂ and Aₙ as well as the side chains A₁^{'}, A₂^{'} and Aₙ^{'} point to the one side (called A-side) and the side chains B₁, B₂ and Bₙ as well as the side chains B₁^{'}, B₂^{'} and Bₙ^{'} point to the other side of the hairpin (called B-side).

This important feature of the invention allows the side chains pointing to one side of the hairpin to be used to stabilize the hairpin structure (stabilizing side), while the side chains on the other side (free side) can be used not only to additionally stabilize the structure but in particular to freely adapt the molecule to the intended use, preferably to mimic a naturally occurring molecular structure, in particular a protein domain, e. g. a protein binding site. Either the A or the B side forms the stabilizing side.

As two or three interaction pairs on the stabilizing side are enough to stabilize the hairpin structure in a longer peptide (m ≥ 1) the remaining side chains on the stabilizing side can also be used to mimic a structural motif or molecular structure, e. g. a peptide binding region. Additionally the stabilizing side chains can even contribute to mimic such a structural motif. This advantageously allows using the compounds according to the invention as some kind of molecular adapter, which has on one side (A or B) a binding site for structure 1 (e. g. protein 1) and on the other side a binding site for structure 2 (e. g. protein 2). This way the compounds according to the invention can be used to bring two molecular structures that are normally not close to each other into close proximity.

Advantageously the structure formation of the compounds according to the invention depends more on the position and stereochemistry of the substituents than on their chemical nature or size. Consequently the hairpin-like structure formation and the formation of the above mentioned A-side and B-side is pretty much independent of the chemical nature and size of the substituents as long as the following requirements on the position and stereochemistry of the substituents are met:
- the oligomers mainly consist of heterochiral β-amino acid building blocks, which are either uniformly in R,S or S,R conformation,
- the oligomers contain at least two, preferably three interacting pairs stabilizing the hairpin structure,
- almost all side chains have at least two non-hydrogen atoms.

The side chains at the C_{α} and C_{β} positions of the β-amino acid building blocks that have at least two non-hydrogen atoms advantageously lock the central torsion of both β-peptide oligomers in a *s-trans* conformation as shown in the Formula 2 - see b) for Newman projection - for the residue n (which can also be applied to residue 1, 2, 1', 2' and n'):

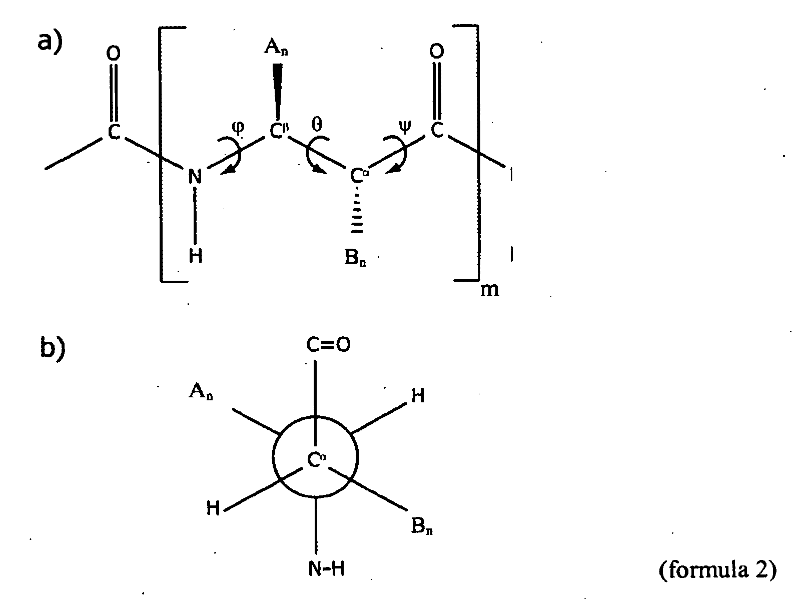

Formula 2 a) shows the residue n in the heterochiral β^{2,3}-amino acid N-terminal oligomer and the torsion angles ϕ, θ and ψ of the β-peptide backbone. Formula 2 b) shows the Newman projection of residue n along the central torsion angle θ.

The backbone of each oligomer is preferably a linear β-peptide backbone, thus the carbon atoms of the backbones are preferably not part of a ring structure (The backbone in the turn motive T can be part of a ring structure). The oxygen atoms in the carbonyl moieties in the β-peptide backbone might be partly or totally replaced by sulfur atoms

Advantageously the backbones of the N-terminal and the C-terminal oligomers are close enough so that hydrogen bonds between the backbones (N-H and O) of the two oligomers can form. These hydrogen bonds form preferably between at least two non-prime residues and the correspondent prime residues:
- backbone of residue 1 (N-H in the N-terminus NT or O) with backbone of residue \1' (O in the C-terminus or N-H),
- backbone of residue 2 (N-H or O) with backbone of residue 2' (O or N-H) and
- backbone of residue n (N-H or O) with backbone of residue n' (O or N-H).

Preferably, the compound according to the invention contains 2 to 8, most preferred 4 to 6 hydrogen bonds between the backbones of the side chains.

One possible arrangement of hydrogen bonds is listed in formula 3 (hydrogen bonds marked by dashed lines):

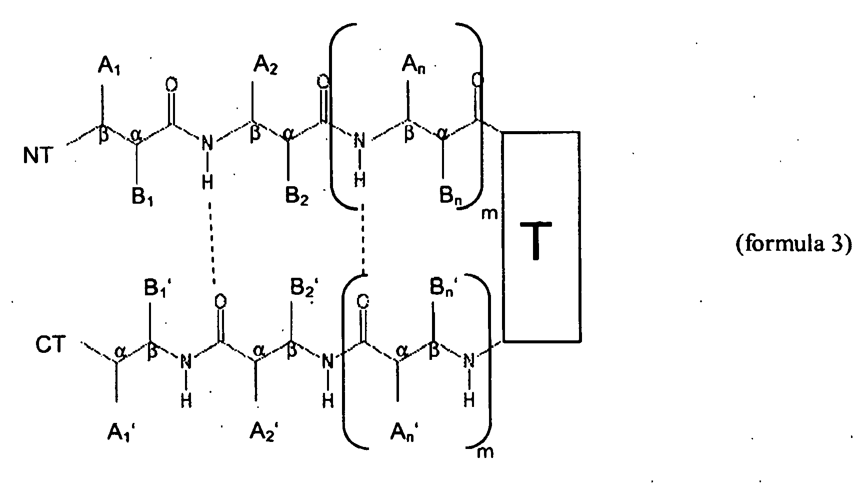

NT is the N-terminus, which is either the free N-terminal amino group or a modification of the N-terminal amino group with the general formula NR₁R₂. CT is the C terminus, which is either the free C-terminal carboxyl group of the C-terminal amino acid or a modification of the C-terminal carboxyl group, preferably with the general formula COR₃ or CSR₃.

In case of a free N-terminal amino group or either one of the R₁ and R₂ being hydrogen an additional hydrogen bond between NT and CT may form.

Another possible arrangement of the hydrogen bonds is listed in formula 4 (hydrogen bonds marked by dashed lines):

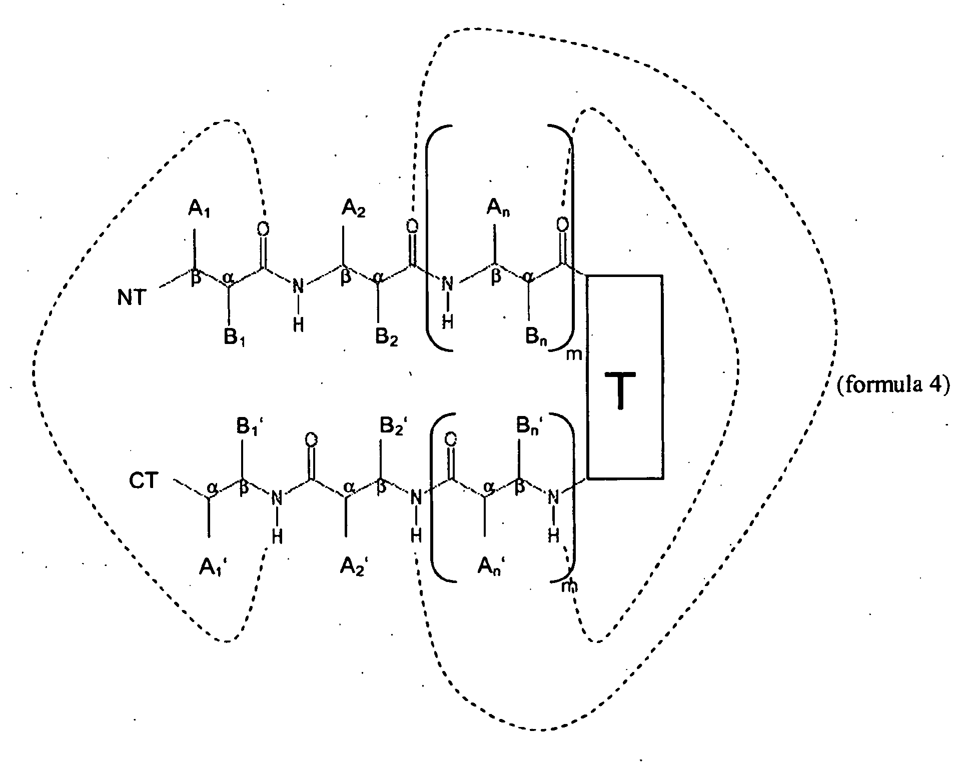

In the following, the side chain interactions are explained in more detail.

Side chains which interact via polar forces include according to the invention polar or charged groups or π-systems.

Preferred polar or charged groups are chosen from amide, carboxyl, hydroxyl, thiol, keto, formyl, amine, imine, phosphate, phosphonate, sulfate, sulfonate, nitro, nitroso, nitrile, guanido and carboxyamido.

Preferred groups with π-systems include one or two aromatic rings (with 5 to 9 C-atoms each) and are preferably chosen from phenyl, benzyl, imidazol, pyrrol, pyrazol, pyridin, pyrimidin, indol, naphtyl, anthryl, phenanthryl, pteridinyl, purinyl, pyranyl, pyrazinyl, pyrazolyl, pyridazyl, pyridyl, pyrimidinyl, quinazolinyl, quinolyl, isoquinolyl, thienyl, furyl, indolyl and isoindolyl.

Examples of stabilising pairs interacting via polar forces are:
(i) 4-Hydroxyphenyl-methyl residue (α-Tyrosine side chain) and 3-(diaminomethylidene amino) propyl residue (α-arginine side chain) forming a cation-π-interaction,
(ii) 4-Hydroxyphenyl-methyl residue (α-Tyrosine side chain), or alternatively a glutamine or threonine side chain and 4-amino-butyl residue (a-Lysine side chain) forming a hydrogen bond,
(iii) 2-Carboxy-ethyl residue (α-glutamate residue) and 4-amino-butyl residue (α-Lysine side chain) forming an ionic interaction (salt bridge).

The polar interaction is preferably a direct interaction between the residues. In aqueous solution water molecules may (additionally) contribute to or mediate the interaction.

Side chains to interact via nonpolar (or hydrophobic) forces preferably consist only of carbon and hydrogen atoms and are preferably chosen from aliphatic or aromatic side chains with 1 to 10 C-atoms.

Nonpolar aromatic and aliphatic side chains may include one hetero atom like nitrogen, sulfur or oxygen. Nonpolar aliphatic side chains preferably do not contain hetero atoms. Preferred nonpolar aliphatic side chains are chosen from ethyl, propyl, isopropyl (α-Valin side chain), butyl, 1-methyl-propyl or sec-butyl(α-isoleucine side chain), *tert*-butyl, isobutyl (α-leucine side chain), pentenyl, tert-pentenyl, neo-pentyl, isopentenyl and hexanyL Preferred nonpolar aromatic side chains are chosen from phenyl, phenylmethyl (α-phenylalanine side chain), benzyl, substituted (preferably with methyl or F, Cl, Br, I) phenyl or benzyl, indolyl (α-Tryptophane side chain), pyrrol, pyrazol, pyridin, pyrimidin, indol, naphtyl, anthryl, phenanthryl, pteridinyl, purinyl, pyranyl, pyrazinyl, pyrazolyl, pyridazyl, pyridyl, pyrimidinyl, quinazolinyl, quinolyl, isoquinolyl, thienyl, furyl, and isoindolyl Examples of stabilising pairs interacting via nonpolar forces are:
(i) a *sec*-butyl residue (α-isoleucine side chain) and a isopropyl residue (α-leucine side chain) or
(ii) two (1*H-*indol-3-yl) residues (α-tryptophan side chain).
(iii) two benzyl residues (α-phenylalanine side chains)

Preferably, the residues 1 and 1', 2 and 2' and 3 and 3' form interacting pairs, either with their side chains on the A side or with the side chains on the B side. The interacting side chains in residues 1 and 1' are preferably polar side chains, preferably forming a cation-π-interaction, most preferred between a Tyrosine- and an Arginine-side chain, e. g. B1 being Y (Tyrosine-side chain) and B1 being R (Arginine-side chain). The interacting side chains in residues 2 and 2' are preferably non-polar side chains, preferably forming a van der Waals interaction, most preferred between Valine-, Isoleucine-, Leucine-side chains, e. g. B2 being I (Isoleucine side chain) and B2' being L (Leucine side chain). The interacting side chains in residues 3 and 3' are preferably polar side chains, preferably forming a hydrogen bond, most preferred between a hydrogen which is part of a hydroxyl group or amino group, and a carbonyl which is part of a carboxyl or carboxyl amid group, e. g. B3 being K (Lysine side chain) and B3' being Q (Glutamine side chain) - refer also to table 1 for one letter nomenclature.

The remaining side chains, which are not part of these hairpin stabilising pairs, are completely variable. However, almost all of them (all or all but m - 1 per oligomer) contain at least 2 non-hydrogen atoms, preferably two C-atoms. These variable side chains can be either side chains occurring in natural α-amino acids or artificial. Preferably the variable side chains are chosen from the side chains listed in table 1.

Advantageously these variable side chains can be modelled regarding the intended use of the compound of the invention (e. g. to fit into a target protein binding site).

A turn motive T is a group, which leads to an abrupt turn of the polypeptide chain of about 180° in the opposite direction. The turn motive used in the invention preferably does not contain naturally occurring α-amino acid building blocks. They are preferably selected from the groups comprising:
(i) two mono-substituted β-amino acid residues forming a dipeptide sequence, preferably being a (S)-β²-(S)-β³-peptide or an (R)-β²-(R)-β³-peptide,
(ii) two amino acid residues (preferably of α, β or γ amino acids) forming a dipeptide sequence, whereas the backbone of each amino acid is part of a 5, 6 or 7-membered ring or a ring structure comprising 5, 6 or 7-membered rings, the amino acid residues being α-amino acid residues, β-amino acid residues or γ-amino acid residues,
(iii) a ring system containing one, two or three 5 to 7 membered aliphatic or aromatic rings.

Several turn motives which can be applied in the compound of the invention are listed in Suat Kee, K., Current Pharmaceutical Design, 2003, 9, 1209-1224, like a lactam bridge, a bislactam ring, Spirolactam, m-(aminomethyl)benzoic acid (Mamb), azacyclodecenone, amino-carboxy-diaza-cyclodecadione, cyclooctadiene derivatives, nitrophenylthiosemicarbazide, aminopropynyl-aniline, oxazolopiperidinones, bicyclic turned dipeptide (BTD), tendamistat, Benzodiazepines, Azabicyclo amino acid, Endo-bicycloheptendicarbonyl, Azodicarbonyl derivatives, bicyclic diketopiperazine, tricyclic diketopiperazine and β-D-Glucose.

Preferred turn motives according to (i) are: 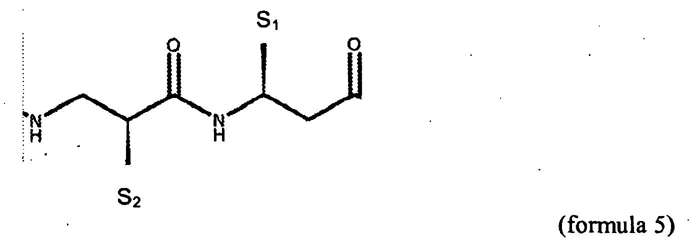 with S₁ and S₂ either in (R,R) or (S,S) configuration and being independently selected from natural or synthetic amino acid side chains, preferably one being nonpolar (e. g. selected from V, L, M, I, F, W) and the other polar, negatively or positively charged (e. g. selected from K, Y, C, S, T, R, N, D, E, Q).

A preferred example being:

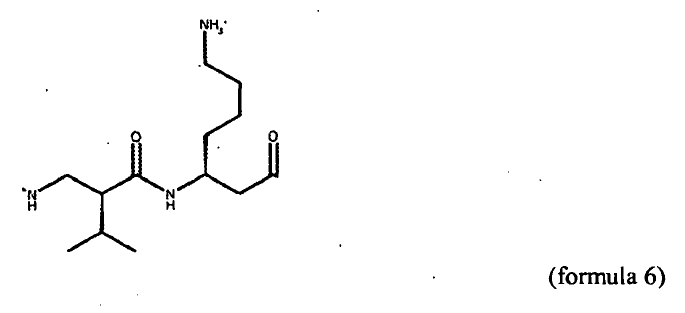

As in the turn motives according to (ii) the peptide backbone is part of a ring or a ring structure is fixed in a turn inducing conformation.

Preferred turn motives according to (ii) are:

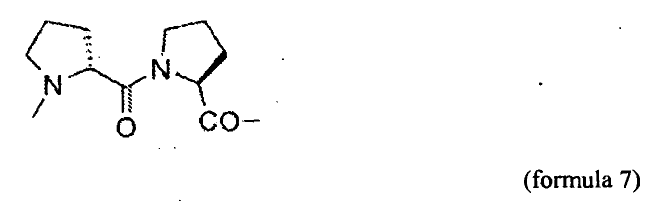

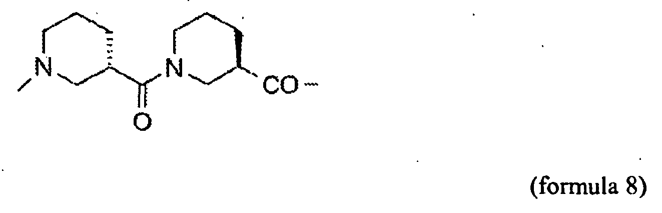

Further preferred turn motives according to (ii) are chosen from formula 9 with k being 0, 1 or 2 and R4, R5, R6, R7 and R8 independently selected from hydrogen and C1 to C3 alkyl:

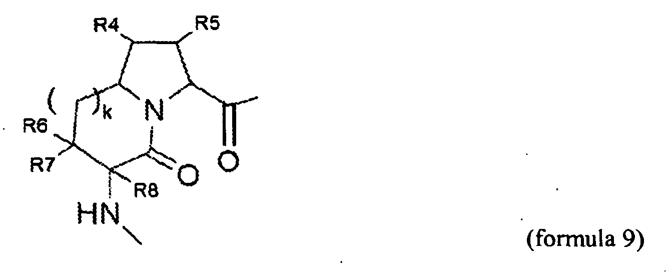

Other preferred turn motives according to (ii) are chosen from formula 10 to 19 with q being 0, 1 or 2 and R9 and R10 being independently selected from natural or synthetic amino acid side chains:

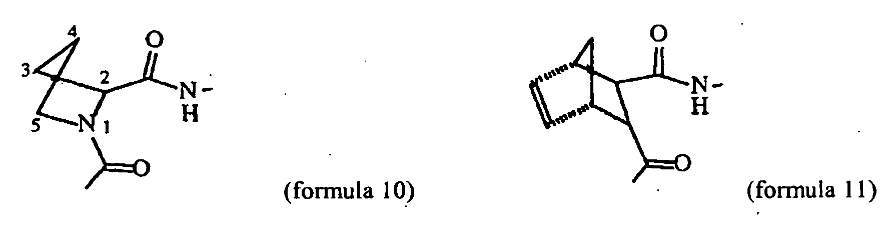

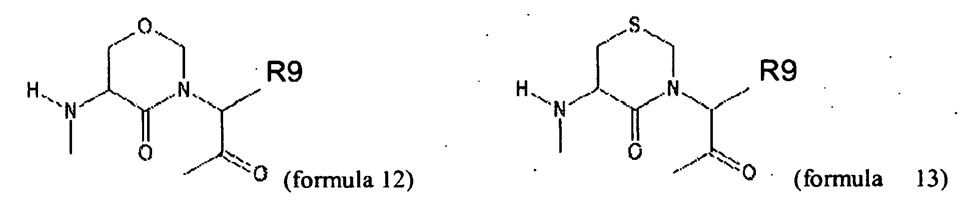

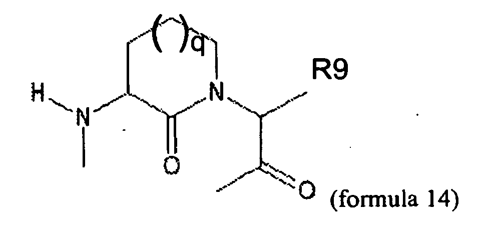

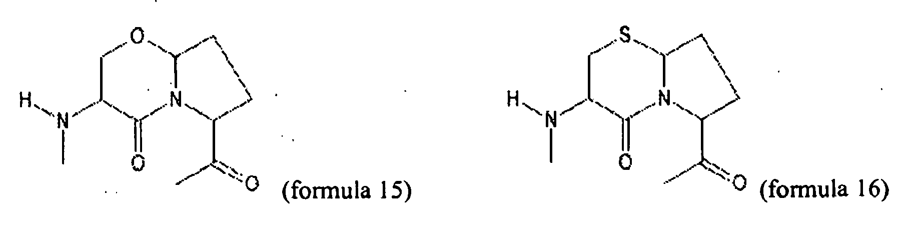

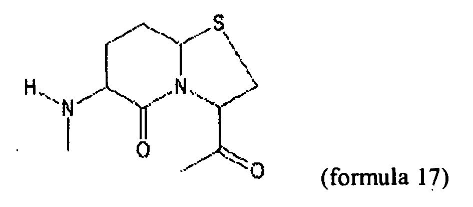

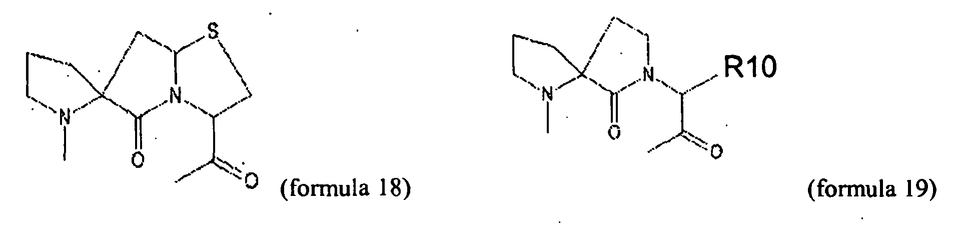

Examples for turn motives according to (iii) are chosen from formula 20 and 21: 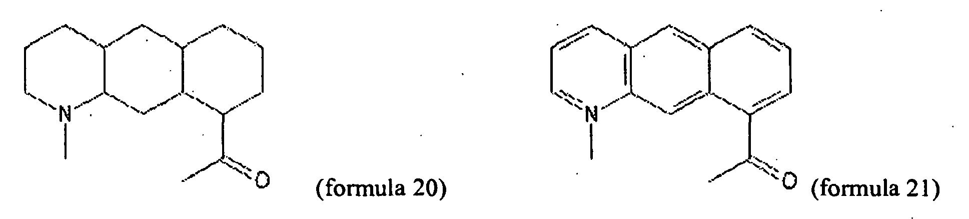 wherein the aliphatic or aromatic rings might be substituted with halogen atoms, polar groups (like hydroxyl or amino groups) or short (1 to 4 C-atoms) aliphatic side chains (alkyl, alkenyl or alkoxy).

NT is the N-terminus, which is either the free N-terminal amino group or a modification of the N-terminal amino group with the general formula NR₁R₂. NT= NR₁R₂, whereas R₁ and R₂ are independent from each other preferably selected from hydrogen or the groups comprising:
(i) a β-amino acid or a chain of two or three β-amino acids;
(ii) a straight chain, branched, cyclic or heterocyclic alkyl group, such as methyl, ethyl, n-propyl, isopropyl, n-butyl, isobutyl, cyclohexyl;
(iii) a straight chain, branched, cyclic or heterocyclic alkanoyl group, such as acetyl or methanoyl (formyl), propionyl, n-butyryl, isobutyryl, pentanoyl, hexanoyl, or cyclohexanoyl;
(iv) a reporter group, such as fluorescein, Alexa488 or biotin, or a reporter protein, such as luciferase or streptavidin or fluorescent proteins such as GFP, YFP, CFP or DsRed;
(v) a fusion tag, such as polyhistidine, Glu-Glu, glutathione S transferase (GST), thioredoxin, protein A, protein G, an immunoglobulin heavy chain constant region (Fc), maltose binding protein (MBP), human serum albumin;
(vi) together with R₃ (see underneath) a bridge between the N- and C-termini thereof to obtain a cyclic peptide. The N- and C-termini can be either connected directly via a peptide bond (R₃ and either R₁ or R₂ just being a covalent bond) or via a linker e. g. based on a guanidine, ethylene glycol oligomers, 2,4-diaminobutanoic acid, 2,3-diaminipropionic acid, 2,2'-diaminopimelic acid, desmosin, or isodesmosine.

CT is the C terminus, which is either the free C-terminal carboxyl group of the C-terminal amino acid or a modification of the C-terminal carboxyl group, preferably with the general formula COR₃ or CSR₃ (R₃ replacing the hydroxyl group of the last amino acid). R₃ is preferably selected from the groups comprising:
(i) a beta-amino acid or a chain of two or three beta-amino acids;
(ii) hydroxyl (COR₃ being a free carboxyl, CSR₃ being thiocarboxyl), alkyl (COR₃ or CSR₃), alkoxy or sulfanyl (COR₃ or CSR₃ being an ester, mono- or dithioester), an amine (COR₃ being an amide or an imide, CSR₃ being an thioamide or an thioimide, C);
(iii) together with R₁ or R₂ a bridge between the N- and C-termini thereof to obtain a cyclic peptide
(iv) a reporter group, such as fluorescein, Alexa488 or biotin, or a fluorescent proteins such as GFP, YFP, CFP or DsRed
(v) a fusion tag, such as polyhistidine, Glu-Glu, glutathione S transferase (GST), thioredoxin, protein A, protein G, an immunoglobulin heavy chain constant region (Fc), maltose binding protein (MBP), human serum albumin.

As the compounds according to the invention contain oligomers of β-amino acids they can be characterized by a special nomenclature for β-peptides using a three letter code for each β-amino acid, with:
first letter = B indicating the β-amino acid;
second letter indicating the side chain of the C_{β} atom and
third letter indicating the side chain of the C_{α} atom. The peptide sequence is written from the N-terminus to the C-terminus.

Applying this nomenclature to the compounds according to the compounds according to the invention the general formula 1 is written as follows:

NT-BA₁B₁-BA₂B₂-(BAₙBₙ)ₘ-T-(BBₙ^{'}Aₙ^{'})ₘ-BB₂^{'}A₂^{'}-BB₁^{'}A₁^{'}-CT (formula 1')

Thus in case m = 2 - meaning the N-terminal oligomer (left to the turn) and the C-terminal (right to the turn) oligomer each comprises four β-amino acids - you get:

NT-BA₁B₁-BA₂B₂-BA₃B₃-BA₄B₄-T-BB₄^{'}A₄^{'}-BB₃^{'}A₃^{'}-BB₂^{'}A₂^{'}-BB₁^{'}A₁^{'}-CT

In the previous text and the following examples of β-peptides according to the invention the second and third letter indicating the side chain are chosen from the table I (column 1):

**Table 1:**

| Side chain abbreviation | Side chain | Corresponding natural occurring α-amino acid bearing the side chain |
|---|---|---|
| A | CH₃- | Alanine |
| R | H₂N-C(=NH)-NH-[CH₂]₃- | Arginine |
| N | H₂N-CO-CH₂- | Asparagine |
| D | HOOC-CH₂- | Aspartic acid |
| C | HS-CH₂- | Cysteine |
| E | HOOC-[CH₂]₂- | Glutamic acid |
| Q | H₂N-CO-[CH₂]₂- | Glutamine |
| G | H- | Glycine |
| H | 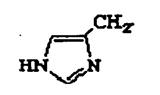 | Histidine |
| I | C₂H₅-CH(CH₃)- | Isoleucine |
| L | (CH₃)₂CH-CH₂- | Leucine |
| K | H₂N-[CH₂]₄- | Lysine |
| M | CH₃-S-[CH₂]₂- | Methionine |
| F | C₆H₅-CH₂- | Phenylalanine |
| S | HO-CH₂- | Serine |
| T | CH₃-CH(OH)- | Threonine |
| W | 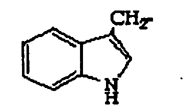 | Tryptophan |
| Y | 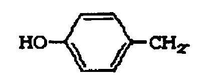 | Tyrosine |
| V | (CH₃)₂CH- | Valine |
| U | HSe-CH₂- | Selenocysteine |
| 1 | CH₂(OH)-CH₂-CH(CH₃)- | |
| 2 | CH₃-CH(OH)-CH₂- | |
| 3 | CH₃-CH₂- | |
| 4 | O₃PO-CH₂- | Phosphoserine |

Thus, as can be seen from table 1, side chains which occur in natural α-amino acids are characterized by the standard one letter code, which is commonly used for α-amino acids. All β-amino acids are di-substituted unless they contain a G in one of the three letters code, in which case they are mono-substituted, i.e. they contain a Glycin "side chain", which is a single hydrogen atom. A β-amino acid, which is mono-substituted on the C_{α} (G as second letter) is also referred to as β²-amino acid. A β-amino acid, which is mono-substituted on the C_{β} (G as third letter) is also referred to as β³-amino acid.

Preferred β-peptides according to the invention and the above mentioned nomenclature have the general sequence:

NT-BA₁B₁-BA₂B₂-BA₃B₃-BA₄B₄-T-BB₄^{'}A₄^{'}-BB₃^{'}A₃^{'}-BB₂^{'}A₂^{'}-BB₁^{'}A₁^{'}-CT

wherein
NT is the N-terminus chosen from the list stated above, preferably NH₂,
A₁ is variable, preferably selected from table 1 and most preferred a nonpolar amino acid side chain, e. g. W or V,
B₁ is an nonpolar amino acid side chain, preferably chosen from V, I, L and M, or selected from methyl (A) and hydrogen (G);
A₂ is variable, preferably selected from table 1 and most preferred a nonpolar or a positively charged amino acid side chain, e. g. L, R, K, W or 3;
B₂ is an aromatic amino acid side chain, preferably chosen from Y, F, H and W, or alternatively selected from methyl (A) and hydrogen (G);
A₃ is variable, preferably selected from table 1 and most preferred a polar or nonpolar amino acid side chain, e. g. T, V, Q, or 2;
B₃ is a nonpolar amino acid side chain, preferably chosen from V, I, L and M;
A₄ is variable, preferably selected from table 1 and most preferred a polar or nonpolar amino acid side chain, e. g. Q, S, 1 and V;
B₄ is a positively charged amino acid side chain, preferably chosen from K and R;
T is a turn motive chosen from the list stated above, preferably BGV-BKG,
B₄' is a negatively charged amino acid side chain, preferably chosen from D and N or a polar side chain, preferably chosen from Q and E;
A₄' is variable, preferably selected from table 1 and most preferred a polar or nonpolar amino acid side chain, e. g. Q, S, 1 and V;
B₃' is a nonpolar amino acid side chain, preferably chosen from V, I, L and M;
A₃' is variable, preferably selected from table 1 and most preferred a polar or nonpolar amino acid side chain, e. g. T, V, Q, or 2;
B₂' is a cationic amino acid side chain, preferably chosen from K, R and H
A₂' is variable, preferably selected from table 1 and most preferred a nonpolar or a positively charged amino acid side chain, e. g. L, R, K, W or 3;
B₁' is an nonpolar amino acid side chain, preferably chosen from V, I, L and M, or selected from methyl (A) and hydrogen (G);
A₁' is variable, preferably selected from table 1 and most preferred a nonpolar amino acid side chain, e. g. W or V,
CT is the C-terminus chosen from the list stated above, preferably COOH or CONH₂.

in these preferred β-peptides the B₁ side chain interacts with the B₁' side chain via non polar forces (van der Waals forces). Preferably the aromatic B₂ side chain interacts with the positive B₂' side chain via a Cation-π-interaction, the non-polar B₃ side chain interacts with the non-polar B₃' side chain via non-polar van der Waals forces and the side chain B₄ interacts with B₄' either via ionic interaction (salt bridge between a positively charged and a negatively charged side chain) or via a hydrogen bond.

Particularly preferred β-peptides according to the invention and the above mentioned nomenclature are selected from the sequences listed in table 2:

**Table 2:**

| β-peptide sequence | name |
|---|---|
| BWA-BLY-BLI-BLK-BGV-BKG-BQI-BLI-BRI-BAW | HP-1 |
| BWA-BLY-BTI-BQK-BGV-BKG-BQH-BLK-BRY-BAW | HP-2 |
| BWA-BRY-BVI-BQK-BGV-BKG-BQH-BLK-BRY-BAW | HP-3 |
| BWA-BKY-BVI-BQK-BGV-BKG-BQH-BLK-BRY-BAW | HP4 |
| BWA-BTY-BVI-BQK-BGV-BKG-BQH-BLK-BRY-BAW | HP-5 |
| BVI-BWY-BVI-BQK-BGV-BKG-BQH-BLK-BRY-BAW | HP-6 |
| BVI-BWA-BVI-BQK-BGV-BKG-BQH-BLK-BRY-BAW | HP-7 |
| BVI-BWY-BQI-BSK-BGV-BKG-BQH-BLK-BRY-BAW | HP-8 |
| BVI-BWY-BQI-BSK-BGV-BKG-BQH-BLK-BRF-BAW | HP-9 |
| BVI-BWY-BQI-BSK-BGV-BKG-BQH-BLK-BRA-BAW | HP-10 |
| BVI-BWY-BQI-BSK-BGV-BKG-BQH-BLK-BRV-BAW | HP-11 |
| BVI-BWY-BQI-BSK-BGV-BKG-BQH-BLK-BRI-BAW | HP-12 |
| BVI-BWY-BQI-BSK-BGV-BKG-BQH-BLK-BRL-BAW | HP-13 |
| BVI-BWY-BQI-B1K-BGV-BKG-BQH-BLK-BRI-BAW | HP-14 |
| BVI-BWY-BQI-B1K-BGV-BKG-BQH-BLK-BRI-BAW | HP-15 |
| BVI-BWY-B2I-BVK-BGV-BKG-BQS-BLK-BRY-BVI | HP-16 |
| BWI-B3Y-B2I-BVK-BGV-BKG-BQS-BLK-BRY-BVI | HP-17 |

In the above listed sequences the underlined sequence BGV-BKG represents the turn motive T according to formula 5 (a β²,β³-dipeptide), which is either in (S)-(S)- or (R)-(R)-configuration. Further examples according to the invention are sequences, wherein BGV-BKG is replaced by another turn motive as listed above.

All di-substituted β-amino acids are uniformly heterochiral and either in (R,S)- or (S,R)-configuration. As far as the β-peptides include further mono-substituted β-amino acid (as BGK in HP-3) they are either in the (R)- or (S)-configuration.

In the above listed examples the N-terminal oligomer (left to the turn) and the C-terminal (right to the turn) oligomers consist each of four β-amino acids (m = 2).

In certain embodiments, the compounds of the invention (including the above listed β-peptides) may further comprise modifications analogous to post-translational modifications. Such modifications include, but are not limited to, acetylation, carboxylation, glycosylation, phosphorylation, lipidation, and acylation. As a result, the modified polypeptides may contain non-amino acid elements, such as polyethylene glycols, lipids, poly- or monosaccharide, and phosphates.

Advantageously the compounds according to the invention mimic β-hairpin structures.

β-hairpins together with α- and polyproline-II (PPII) helices are the protein structural frameworks most commonly used in protein recognition of cellular signalling and, therefore, of great pharmacological interest.

The compounds according to the invention, which are designed β-hairpin like structures that mimic natural β-hairpins and/or loops of proteins with essential implications in molecular recognition processes, have a large number of potential applications: redesign of proteins containing β-sheets, excising a binding site from a larger protein domain, design of binding sites for unique ligands or set of ligands, design of mini-proteins for targeting protein ligands and DNA, develop biosensors (metal binding sites), *de novo* design of catalysts, etc..

One very interesting and representative example for cell signaling protein-protein interactions that can be mimicked by the compounds according to the invention mediated by β-hairpin is the recognition of proline-rich sequences (PRS) by WW domains, the smallest poly-proline II (PPII) fold recognizing domain: The naturally occurring WW domains consist of about 40 residues that form a three stranded β-sheet. They are characterized by two conserved Tryptophane residues (W in the one letter code), which are found in almost all WW domains (Sudol, M et al. Cell 2000, 103, 1001; Trends Biochem. Sci. 1996, 21, 161; Exp. Mol. Med. 1996, 28, 65; Macias, M. J. et al. FEBS Lett. 2002, 513, 30; Nature 1996, 382, 646). One example of a protein with a WW domain is the human Yes associated protein YAP65, which exhibits a WW domain, recognizing the sequence motif PPXY in a PPII fold (thus being a type I WW domain). YAP65 is a transcriptional co-activator and for instances influencing TGF-beta signaling, thus playing a role in some cancers.

WW domains are also found in proteins implicated in Ras or MAP kinase signaling pathways and in ligands that bind to transcription factors.

In one embodiment the compound according to the invention mimics the recognition site for PPII (binding center) of a WW domain. In this embodiment the compound according to the invention carries two tryptophan-side chains (W as listed in table 1). One of the W-side chains is preferably located next to the N-terminus, thus preferably one of the side chains chosen from A₁ and A₂ or B₁ and B₂ is W. Alternatively NT contains at least a further β-amino acid bearing a W side chain. The second W-side chain is preferably located next to the C-terminus, thus preferably one of the side chains chosen from A₁ and A₂ or B₁ and B₂ is W. Alternatively CT contains at least a further β-amino acid bearing a W side chain.

Preferred examples according to the inventions mimicking a WW-domain, preferably from YAP65, are the β-peptides HP1 to HP15, most preferably chosen from HP1, HP2 and HP3.

The interaction between WW-domains and their proline-rich ligands play an important role in many signaling pathways and are implicated in several human diseases, for instance in Liddle's Syndrome, Huntington's disease (high blood pressure), muscular dystrophies (e. g. caused by mutations in the dystrophin molecule), Alzheimer's disease, virus infections and neurological disorders.

As the compounds according to the invention are able to mimic WW-domains and other β-hairpin structures, they can be used to mimic or modulate the interactions between those molecules (structures) and their ligands. The action (e. g. binding to the proline-rich ligand) can be either as antagonist or agonist.

Another object of the invention is therefore the use of the compounds of the invention in pharmacy or biotechnology or as research tool in science, in particular to mimic a protein domain or a protein binding domain. The compounds of the invention advantageously can be used to promote or inhibit the interaction of proteins and to modulate signaling pathways, in which the interaction of a β-hairpin structure plays a role.

A further object of the invention is a pharmaceutical composition containing at least one compound according to the invention and the use of the compounds according to the invention in medicine, in particular to treat Liddle's Syndrome, Huntington's disease, high blood pressure, muscular dystrophies (e. g. caused by mutations in the dystrophin molecule), Alzheimer's disease, virus infections and neurological disorders.

### Examples:

Without further description, it is believed that a person of ordinary skill in the art can, using the preceding description and the following illustrative examples, make and utilize the compounds of the present invention. The invention is further described, by way of illustration only, in the following examples which refer to the accompanying figures in which
**Fig. 1** represents the exemplary β-peptide hairpins HP1 (a), HP2 (b), and HP3 (c) which consist of three main elements, the turn motive and the sides A and B. Refer to Table 1 for the three letter code.
**Fig. 2** a to d represent different views of the 3D structure of β-peptide hairpin HP3 illustrating the side chains form the A-side and the B-side. **Fig. 2a** representing a side view, with the A-side pointing to the left and the B-side pointing to the right, **Fig. 2b** representing a view facing the A-side, **Fig. 2c** representing the "bottom view", facing the open N- and C-termini of the hairpin, with the A-side pointing to the left and the B-side pointing to the right, and
**Fig. 2d** representing the "top view", facing the turn motif, with the A-side pointing to the left and the B-side pointing to the right.

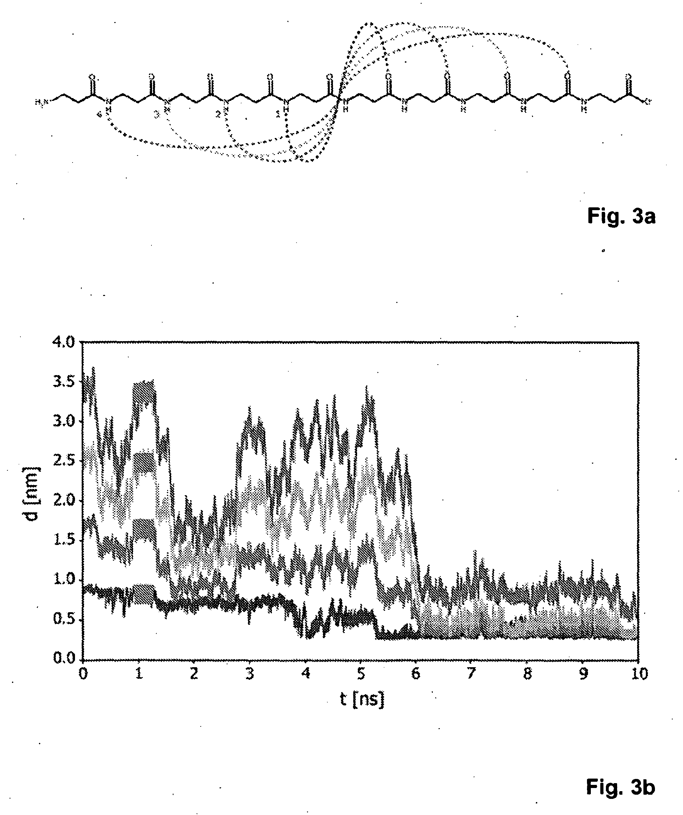

**Fig. 3** **a** and **b** illustrate hydrogen bond formation in the backbone of the β-peptide hairpin HP1. Fig. 3 represents the last 10 ns from a 20 ns trajectory of HP1: **Fig. 3a** depicts the forming H-bonds between the amide protons of one oligomer and the carboxyl oxygen atoms of the other oligomer; **Fig. 3b** plots the calculated distance of each proton to its according oxygen atom against time. The decrease of distance between those atoms illustrates how H-bonds are formed rapidly between the backbone atoms of the β-peptide.
**Fig. 4** demonstrates the position of hydrogen bonds in the two most common folding variants of the β-peptide hairpin HP2:

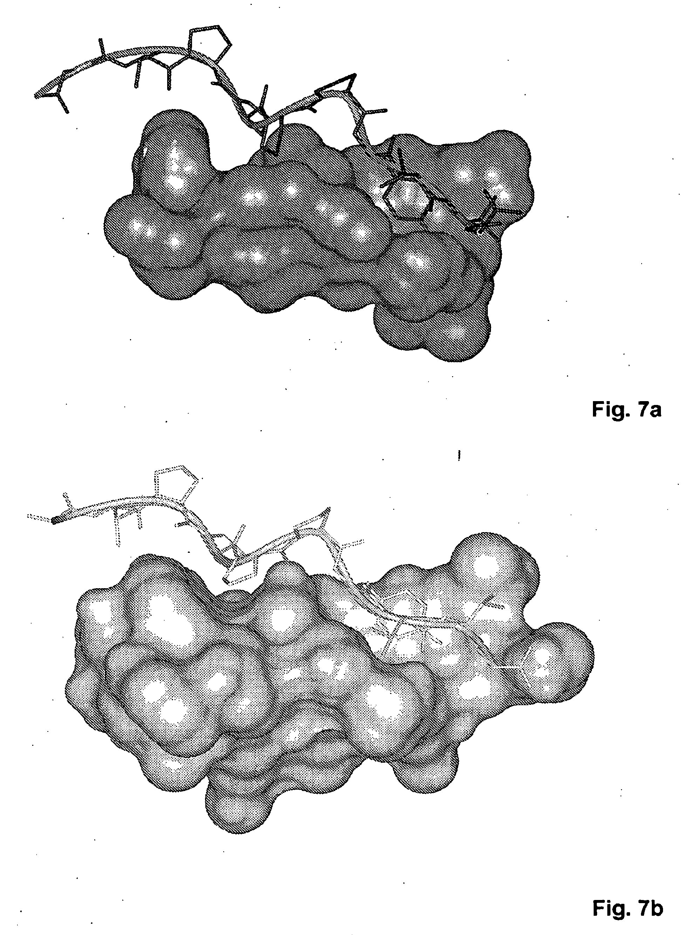

   In **Fig. 4a**, Conformer C₁₀, the conformation, with a 1→2 turn (cluster 1 from Fig. 7) forms H-Bonds between the amide protons of residues 1 (N-terminus), 2, 3 and 4 (all on the left) and the carboxyl oxygens of residues 1' (C-terminus), 2', 3' and 4' (all on the right) and one hydrogen bond in the turn motive.
   In **Fig. 4b** Conformer C₁₂ with a 1←4 turn (cluster 2 from Fig. 7) forms H-Bonds between the amide protons of residues 1', 2', 3' and 4' (all on the right) and the carboxyl oxygens of residues 1, 2, 3 and 4 (all on the left).

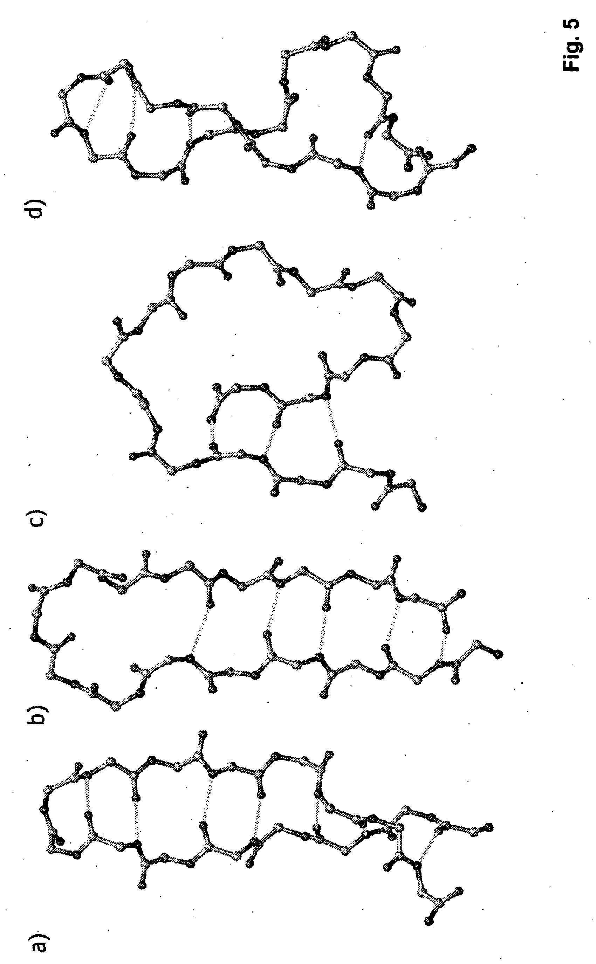

**Fig. 5** depicts some of the many different ways the α-peptide G4 (comparison example) can fold in aqueous solution (snapshots from the 300 K trajectory): a) the desired hairpin conformation (cluster 1); b) a. 'mismatched' hairpin (cluster 2); c) a 'curled' conformer (cluster 3); d) a 'mismatched' hairpin with a g-type turn around the D-Pro (cluster 4).

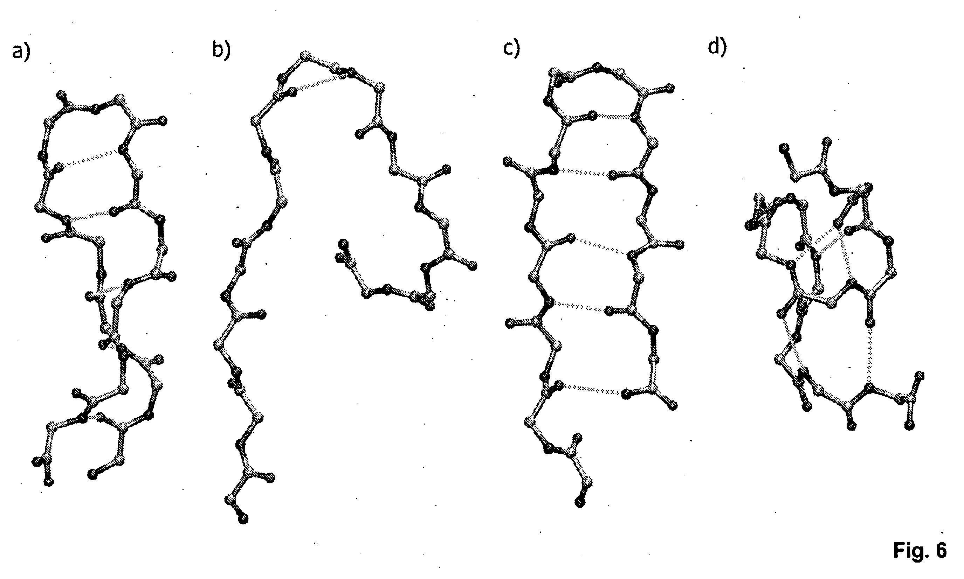

**Fig. 6** depicts several possible secondary structures the α-peptide Trpzip2 (comparison example) can fold into in aqueous solution (snapshots from the 300 K trajectory): a) the 'productive'desired hairpin conformation; b) no formation of a hairpin structure, but forming of a γ-turn at the Gly residue; c) a 'mismatched' hairpin; d) a distorted helical conformation.
**Fig. 7** shows the mimicking of the interaction of a naturally occurring α-peptide, namely strands 2 and 3 of the YAP65 WW-domain residues 27 to 39 (**Fig. 7b**) from PDB 1JMQ with the PPII Ligand, using the β-peptide HP3 **(****Fig. 7a****).** In both figures (**7a and 7b**) only the side chains and a backbone ribbon of the PPI- ligand is shown. In **Fig. 7a** the β-peptides HP3 and in
**Fig. 7b** the α-peptidic YAP65 WW-domain is shown as a sphere. The comparison between the interaction of the α-peptide with its ligand **(****Fig. 7b****)** and the synthetic β-peptide with the same ligand **(****Fig 7a****)** shows great similarities. Clearly visible is the insertion of the two tryptophan side chains (labeled WW) of both molecules into the recognition pocket of the PPII helix.

### Synthesis and Characterization of Compounds

The compounds of the invention may be synthesized by following procedures described for other β^{2,3}-derivatives. Alkylation at the α-position of β³-amino acids and titanium-catalysed addition of ester enolates to *tert*-butanesulfinyl imines (English, E. P. et al. J. Biol. Chem. 2006, 281, 2661) are attractive routes for accessing different diastereoisomers (Scheme 1). Lithiation of *N*-protected β-amino esters by treatment with lithium diisopropylamide (LDA) followed by addition of a variety of alkylating reagents afford preferentially (Heinrich Estermann, D. S. Helvetica Chimica Acta 1988, 71, 1824. Gardiner, J. et al. A. J Org Chem 2004, 69, 3375. Peng, Y. et al. Org Lett 2004, 6, 3781) the stereoisomer matching the absolute configuration established by the modelling studies. The starting β³-aminoacids are either commercially available or easily prepared by Arndt-Eistert homologation of *N*-Boc-protected α-amino acids. Another versatile method for the asymmetric synthesis of β^{2,3}-amino acids consists of the tandem conjugate addition of chiral nitrogen nucleophiles (e.g. lithium 1-phenylethylamine, readily available in both enantiomeric forms) to α,β-unsaturated esters and subsequent trapping of the β-amino ester enolate with electrophiles. (Langenhan, J. M.; Gellman, S. H. J Org Chem 2003, 68, 6440. Michael, J. P et al. Org Biomol Chem 2005, 3, 836. Pu, X.; Ma, D. J Org Chem 2003, 68, 4400. Sewald, N. et al. J Org Chem 1998, 63, 7263.) Alternatively, the enantioselectivity might arise from the use of chiral catalysts or through complexation of the lithium cation with chiral ligands (Sakai, T. et al. J Org Chem 2006, 71, 4706. Taylor, M. S. et al. JACS 2005, 127, 1313). Appropriately N/C-protected β^{2,3}-amino acids may be purified following standard solution methods. Efficient protocols have been developed for the preparation of β-peptides either using solution (Boc-chemistry) or solid-phase synthesis methods (Kimmerlin, T.; Seebach, D. J Pept Res 2005, 65, 229.) (Fmoc-chemistry, Rink amide or Wang resin, Scheme 1). Small peptides have been built in solution by segment-coupling. Long β-peptides can be accessed through the chemical ligation methodology.

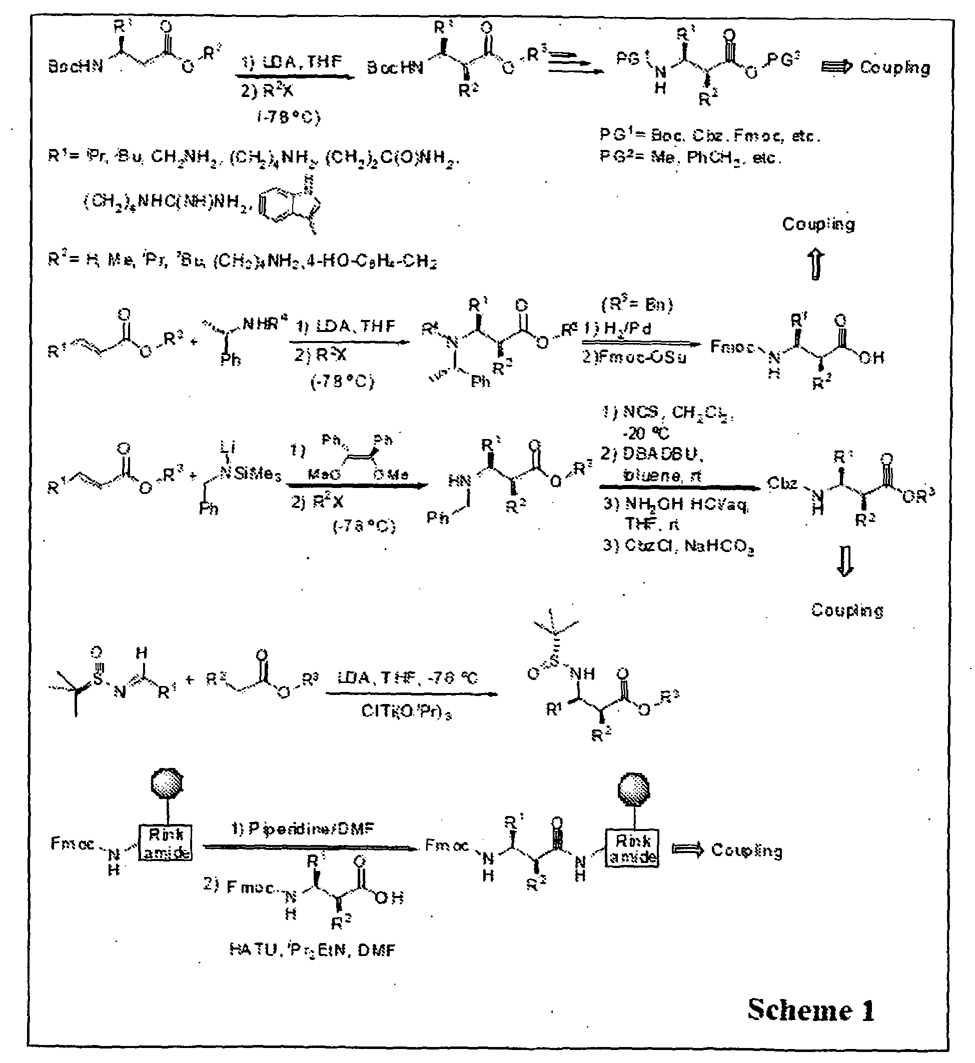

Differing from the general description of the invention in Scheme 1 R¹ is here the side chain on the C_{β}-atom and R² the side chain on the C_{α}-Atom.

All simulations and part of the analysis were carried out with the Gromacs suite of programs (version 3.3.1). (Lindahl, E.; Hess, B.; van der Spoel, D. J. Mol. Model. 2001, 7, 306. Van der Spoel, D. et al. J. Comput. Chem. 2005, 26, 1701.) The Gromos 96 (53a6) force field (Oostenbrink, C. et al. Eur. Biophys. J. Biophys. Lett. 2005, 34, 273; J. Comput. Chem. 2004, 25, 1656) has proven to work well also for β-amino acids and β-peptides (Daura, X. et al. J. Am. Chem. Soc. 2001, 123, 2393; Chem. Eur. J. 1997, 3, 1410; Angew. Chem. Int. Edit. 1999, 38, 236; Proteins: Struct., Func., and Genetics* 1999, 34, 269. Trzesniak, D. et al. Biopolymers 2006, 83, 636). The necessary amount of counter-ions (Cl⁻ and Na⁺) was added to ensure a neutral system. Before the simulation steps, steepest descent energy minimizations and position restrained MD simulations to energy convergence (20 to 100 ps) were performed. The protonation state of the peptide was assumed for pH 7. We studied the folding behavior of the α- and β-peptides unbiased from previous structural investigations. The starting conformations are completely extended in all simulations and at all different simulation temperatures. VMD (Humphrey, W. et al. J. Mol. Graph. 1996, 14, 33.) and POV-Ray were used for visualization.

Sequences of the designed peptide models can be found in Table 3. The structural properties of the β-peptide scaffolds were compared to two representative α-peptides: *i*) G4, an α-peptide designed to form a hairpin and to resemble the binding site of the YAP65 WW domain; (Espinosa, J. F. et al. Biopolymers 2005, 80, 303.) *ii*) Trpzip2, a Tryptophan zipper hairpin known to fold stable (PDB: 1LE1) (Cochran, A. G. et al. Proc. Natl. Acad. Sci. U. S. A. 2001, 98, 5578; Proc. Natl. Acad. Sci. U. S. A. 2002, 99, 9081. Zhang, J. et al. Proteins: Struct., Func., and Bioinformatics 2006, 62, 672.). The sequences of G4 and Trpzip2 can be found in table 3.

The layout of the compound according to the invention combines a structural requisite, the use of heterochiral β^{2,3}-disubstituted β-peptides which forces the two β-peptide oligomers to adopt an extended conformation, with the advantage of a two-sided hairpin structure, both sides of which can be freely design to fit its purposes by choosing the substituents of the β-amino acid building blocks. One side chain of each building block is used to achieve affinity and specificity for binding (A-side; cf. Fig. 1 and 2) while the other forms one half of an interacting pair to stabilize hairpin formation (B-side; cf. Fig. 1 and 2).

The turn-inducing sequence, here the central dipeptide (residues 5 and 6, BGV-BKG) is crucial for the formation of a hairpin-like structure. Stabilization of the hairpin is intended by π-cation interaction between the Tyr side chain of residue 2 and the Arg side chain of residue 9, and by the hydrophobic packing between the aliphatic side chains of residues 3 and 8. The two Trp side chains on side A (cf. Fig. 1) that introduce specificity and affinity by forming a wall of the Pro-Pro recognition pocket further stabilize the fold through π-interactions.

The β³-positions of residues 2 to 4 and the β²-position of residues 7 to 9 are free for substitutions with side chains of virtually any function and chemistry. The range of possible side chain substitutions is only limited by synthetic availability and not limited to the canonical substituents of the α-peptides. Thus, the availability of possible functions and binding sites is enormous and allows side chains and functionalities to be adapted to virtually any needs.

**Table 3: Examples for Peptide sequences**

| **Names** | **Sequence** | **SEQ ID No.** |
|---|---|---|
| *α-peptides as comparative examples* | | |
| G4¹ | RYFLNHVpGKQTTTWQ-NH₂ | 1 |
| Trpzip2 | SWTWENGKWTWK-NH₂ | 2 |
| PP2 | GTPPPPYTVG | 3 |
| *β-eptides² - Examples according to the invention* | | |
| HP1 | BWA-BLY-BLI-BLK-BGV-BKG-BQI-BLI-BRI-BAW | |
| HP2 | BVI-BWY-BQI-BSK-BGV-BKG-BQH-BLK-BRL-BAW | |
| HP3 | B1I-BWY-B2I-BGK-BGV-BKG-BQH-BLK-BRY-BAW | |

| | | |
|---|---|---|
| ¹ The lower case 'p' stands for D-Proline. ² The three letter code for the β-amino acids: 1^{st} letter: B means β-amino acid; 2^{nd} and 3^{rd} letter: Side chain of the corresponding α-amino acid placed on the C_{β}- or C_{α}-atom, respectively, numbers correspond to non-natural side chains, 1 being a 3-Hydroxy-1-methyl-propyl residue and 2 being a 2-Hydroxy-propyl residue - s. table 1 for details. | | |

### Example 1:

The β-peptide HP1 serves as *proof-of-concept* that hairpin formation of rationally designed β-peptides takes place in water. The canonical MD-simulations were performed with a standard setup for 10 ns time. The molecules were solvated in a large dodecahedric box containing about 14,000 SPC water molecules. Thereby, a minimum distance of 1.5 nm between the fully extended solute and the box borders was assured. Periodic boundary conditions were applied. The temperature (298 K) and pressure (1 bar) were weakly coupled following the Berendsen method with time constants of 0.1 and 0.5 ps, respectively (Berendsen, H. J. G.; et al. J. Chim. Phys. 1984, 81, 3684). Coulomb and van der Waals interactions were modeled with a twin range cut-off (0.8 and 1.4 nm). Constraints were applied with the SHAKE algorithm (Miyamoto, S.; Kollman, P. A. J. Comput. Chem. 1992, 13, 952.).

HP1 folds within only 6 ns from extended state to a hairpin-like structure and remains stable as such in a 10 ns canonical MD-simulation (cf. Fig. 3). The folding process occurs in a zipper-like manner nucleated at the turn region (residues 5 and 6), and closing the hydrogen bonds step by step towards the termini. From the formation of the hairpin-like structure at 6 ns simulation and also after extension of the simulation to 20 ns the conformation does not change and remains stable (cf. Fig. 3). Only the terminal residues show certain flexibility. The averaged torsion angles of the strand-like residues 1 to 4 and 6 to 10 (ϕ = -120°, θ = -175°, ψ = 135) are in perfect agreement with data obtained by other groups in *ab initio* MO theory calculations on blocked heterochiral β^{2,3}-amino acids. Employing the same MD approach on G4, no folding event towards a β-hairpin was monitored.

### Example 2:

The *prototype*-peptide HP2 presents polar side chains on side A (Fig. 1b) instead of the mostly aliphatic side chains on the A-side of HP1. Thus, we can prove that the hairpin formation in these β-peptides is not like a hydrophobic collapse induced by side chain hydrophobicity. HP2 combines the functionalities of HP1 that lead to rapid and robust formation of hairpin-like folds (cf. Fig. 1a and 1b) with our first attempts to mimic the PRS binding site of the WW domain. REMD simulations (Hukushima, K.; Nemoto, K. J. Phys. Soc. Jpn. 1996, 65, 1604. Okabe, T. et al. J. Mol. Biol. 2005, 354, 173.) are used to reach the thermodynamically most stable state and to avoid getting trapped in local minima. Due to the parallel simulation at different temperatures and the frequent exchange of conformations (replicas) between them, an enhanced sampling of the conformational space is ensured. To obtain deeper insight into the folding properties of the β-peptide hairpins, and in order to compare with α-peptides, replica exchange MD (REMD) (Hukushima, K.; Nemoto, K. J. Phys. Soc. Jpn. 1996, 65, 1604. Okabe, T. et al. Chem. Phys. Lett. 2001, 335, 435.) simulations at constant pressure (NPT - Seibert, M. M. et al. J. Mol. Biol. 2005, 354, 173.) were performed for HP2, G4, and Trpzip2 (see Table 3 for sequences). The completely extended peptides with all backbone torsion angles set to 180 degrees (with exception of the D-Pro residue of G4) were centered in small dodecahedric boxes of SPC water molecules. Since the exchange probabilities are based on a Metropolis criterion employing the differences in temperature and potential energies of two replicas, the exchange rate depends on the system size. Thus it is crucial for comparability to use similar sized systems and the same temperatures for the replicas. To ensure a good comparability of our β-peptide to the strongly related α-peptide G4, we recalculated the 20 ns trajectory of HP2 (big box) in a box of the same size as for G4. The very similar exchange rates obtained allow direct comparison of the folding properties from the sampling trajectory at 300 K. For the REMD 15 replicas with temperatures of 300, 304, 308, 312, 316, 320, 325, 330, 335, 340, 346, 352, 358, 364, and 370 K were used together with an 'unphysical', not exchanging trajectory at 380 K. The simulations were carried out for 20 ns, meaning an overall 300 ns sampling time for each peptide obtained by the parallel simulation of 15 replicas. A twin range cut-off for van der Waals (0.9/1.4 nm) and a smooth particle mesh Ewald algorithm for Coulomb interactions (switching distance of 0.9 nm) were used. The neighbor lists were updated every 0.01 ps (Essmann, U. et al. J. Chem. Phys. 1995, 103, 8577). Temperature and pressure were kept constant by Berendsen weak coupling (Berendsen, H. J. C.; et al. J. Chem. Phys. 1984, 81, 3684) with coupling constants of 0.1 ps for the temperature and 1 ps for the pressure. Constraints were applied to the bonds of the peptide with the LINCS algorithm. (Hess, B. et al. J. Comput. Chem. 1997, 18, 1463). To analyze the results of the REMD simulations the conformations of the lowest temperature trajectories were clustered by a simple rmsd criterion for the backbone atoms (C, Ca, N, O, and in case of the β-peptide C_{β} additionally, rmsd cut-off 0.1 nm) of the last 15 ns of the sampling trajectories at 300 K, and the development of distances of selected backbone atoms were measured.

The REMD simulations prove the results from the MD simulations of HP1 and provide comparability to the folding behavior and conformational stability between HP2 and the two α-peptide hairpins (G4 and Trpzip2). HP2 shows again the strong folding tendency towards a hairpin-like conformation. Due to the enhanced sampling of REMD with trajectories at higher temperatures (up to 370 K), the sampling trajectory at 300 K pays a first visit to the hairpin-like conformation already after 2 ns simulation time. Once the hairpin is formed, it remains stable for the rest of the simulation time. The conformational features of HP2 are similar to the ones of HP1 discussed above. Due to the reduced side chain hydrophobicity of HP2, the stability of the hairpin-like conformation results from three points: i) backbone hydrophobicity, *ii*) the conformational lock of the central torsion due to the hetero-chiral substituents at C_{α} and C_{β}, leading to the strand-like structures, and *iii*) the tendency of the central motif to form a turn conformation. The observation of the contiguous replicas shows a comparable behavior, while for higher temperatures a folding alternative begins to populate more. Clustering the 300 K trajectory from 5 to 20 ns with an rmsd cut-off of 0.1 nm results in 7 different clusters. Conformer C₁₀ is populated at a rate of around 87%, and the competing conformer C₁₂ at a rate of 7%. There are nearly no folding alternatives and the observed flexibility results from the turn segment.

In conformer C₁₀, the central turn forms a hydrogen bond in forward direction along the sequence (from the NH of residue 5 to the CO of residue 6, 1→2 interaction) closing a 10-membered pseudo-cycle (cf. Fig. 4a). The alternative conformer C₁₂ is characterized by a 12-membered pseudo-cycle and a hydrogen bond from the NH of residue 7 to the CO of residue 4 (cf. Fig. 4b). This turn conformation shows a 1←4 interaction, and is thus a β-peptide variant of an α-peptide β-turn. The conformation of the turn influences the H-bond orientation of the whole hairpin. The C₁₂ conformer features only H-bonds in backward direction, while conformer C₁₀ features only H-bonds in forward direction. The conformer C₁₂ is nevertheless barely populated or absent in the trajectories with lower temperature. The importance of conformer C₁₂ rises only with the temperature, being the highest populated fold during the simulation time of the unphysical trajectory at 380 K. In the sampling trajectory at 300 K only the conformer C₁₀ is found in a considerable amount, with only few alternative folds.

The first appearance of a β-hairpin in the simulation of the α-peptide G4 takes place after 4 ns. There are more drastic structural fluctuations and more alternative folds for G4 than for HP2. Nevertheless, the highest populated conformation among the 18 alternatives (clustering by rmsd with a 0.1 nm cut-off; only 7 for HP2) is the desired β-hairpin (Fig. 4a) with a proportion of about 67%. Alternative folds are mismatched hairpins with non-β-turns represented by e.g. cluster 2 with a small loop (cf. Fig. 5b) and cluster 4, a distorted hairpin with a γ-turn at the D-Pro residue (cf. Fig. 5d). Additionally there is a 'curled' conformation (cluster 3, Fig. 5c).

Trpzip2 is intended as another benchmark for comparison with the folding behavior of HP2. The formation of a hairpin structure in Trpzip2 seems not as favored as for the two previous peptides HP2 and G4. The large accessible conformational space for Trpzip2 with its 60 independent conformers renders clearly the enormous flexibility, especially in comparison to HP2. The highest populated cluster (just 30%) has an rmsd of only 0.088 nm referred to the lowest energy model from the pdb-entry 1LE1 (Cochran, A. G. et at. Proc. Natl. Acad. Sci. U. S. A. 2001, 98, 5578; Proc. Natl. Acad. Sci. U. S. A. 2002, 99, 9081). Alternatively, there are mismatched hairpins with either γ-turns (cluster 2) or β-turns formed by other residues than 5 to 8 as in the original structure. Even helical conformations can be observed, e.g. in cluster 4. Representative examples for the four highest populated clusters are illustrated in Fig. 6.

This demonstrates the strong tendency of the compounds according to the invention to form hairpin-like structures, especially in comparison with α-peptides of similar size. The simulations of HP2 show its robust folding tendency towards a hairpin-like conformation and also its ability to carry functions on one face to obtain affinity and specificity towards other molecules. In contrast to G4 and Trpzip2, HP2 has obviously no alternative folds to hairpin-like conformations.

### Example 3:

### Recognition of proline-rich sequences (PRS) -

The strong tendency of rationally designed β-peptides to adopt hairpin-like conformations in water makes them extremely interesting as scaffolds for the design of molecules of pharmacological interest. The use of β^{2,3}-amino acids in β-peptides according to the invention facilitates not only the formation of the hairpin structure, but advantageously allows the usage of one face to mimic the surface of α-peptide binding domains (Fig. 1 and 2, A-side), while the other face carries side chains that stabilize the β-sheet formation (Fig. 1 and 2, B-side). Thus β-peptide hairpins can be used to specifically tackle protein-protein interactions in cell signaling, i.e. the recognition of proline-rich sequences (PRS) in signal transduction. The WW domain is the smallest of the proline-recognizing domains and thus a good starting point for the design of artificial PRS receptors. WW is a small adaptor protein domain currently considered an interesting target for drug discovery. It is contained in diverse signaling proteins and involved in a great deal of functional diversity, (Ilsley, J. L. et al. J. Cell Signal 2002, 14, 183. Sheng, M.; Sala, C. Annu Rev Neurosci 2001, 24, 1.) from viral budding to muscular dystrophy or neurological disorders. WW-domains (38-40 aa) fold into a three-stranded β-sheet structure and bind to two different motifs in other proteins: Proline-rich (PPII) and phosphorylated-Serine/Threonine-Proline (p-Ser/Thr-Pro) sequences (Macias, M. J. et at. FEBS Lett 2002, 513, 30). They achieve their dual specificity for ligand recognition through a surface that can, in principle, be reduced to a β-hairpin structure. β-hairpin-like synthetic molecules displaying the crucial recognition elements correctly in 3D can be used to mimic specific recognition sites in WW domains.

A reduction of the PRS recognition site of the YAP65-WW domain to a β-hairpin mimicking the strands 2 and 3 fulfills the needs for ligand binding. Employing a rationally designed β-peptide scaffold circumvents the obvious peculiarities connected with α-peptides. Based on the interactions between the YAP65 WW domain strands 2 and 3 and its ligand PP2 (highest probability structure from the NMR ensemble 1JMQ (Pires, J. R. et al. J. Mol. Biol. 2001, 314, 1147.), side A of HP2 was modified with functionalities that enable the mimicry of this interactions and thus the binding of PP2 (Table 3). The A-side of the resulting peptide HP3 (Fig. 1) resembles the PRS binding site of the YAP65 WW domain (Fig. 7). The Trp side chains of residues BWY2 and BAW10 and the Tyr side chain of residue BRY9 form the hydrophobic patch for the recognition of the poly-proline II structure of the PPXY target sequence. Additionally, the imino-function of the Trp side chain of residue BWY2 can satisfy a backbone carboxy function of PP2. Hydroxy and amino functions of B213 and BLK8 of HP3 resemble Thr37 and Lys30 of the YAP65-WW domain, respectively. The Tyr side chain of the peptide PP2 can interact with the aliphatic part of the Lys side chain of residue BLK8 and an H-bond between the hydroxy-functions of Tyr and the His side chain of residue BQH7 further stabilize this complex.

The scaffold HP3 has to be super-imposed manually with the reduced WW domain (Fig. 7) to estimate the correct orientation of PP2 relative to HP3 and obtain the starting conformation for the simulation of the complex. The HP3/PP2 complex remains stable in a 10 ns MD simulation. The highest populated cluster 5 includes 92% of the conformations from the 10 ns trajectory and is present during the whole simulation time..The remaining 8% of possible conformations spread to 131 clusters. The clustering is performed by first fitting all structures to the backbone of the receptor HP3 and then clustering the heavy atoms of the sequence P⁴PPY⁷ with a 0.1 nm cut-off. The stability of the complex and the specific interactions established by PP2 and HP3 make us propose β-peptide hairpins as promising mimics of PRS binding domains.

### Abbreviations:

The following abbreviations and nomenclature of β-amino acids is used in the description of the invention and in the examples.

Nomenclature of mono- or disubstituted β-amino acids: three letter code with first letter = B indicating the β-amino acid, second letter indicating the side chain of the C_{β} atom and third letter indicating side chain of the C_{α} atom. The letters indicating the side chains are chosen according to the standard one letter code of α-amino acids (see table 1).
- MD: Molecular Dynamics
- NPT-MD: MD which assumes a fixed number of particles, N, constant pressure, P, and temperature, T.
- PDB: Protein Databank
- POV-Ray: Persistence of view raytracer Software
- REMD: Replica exchange molecular dynamics
- SPC water: refers to the simple point of charge model of water molecules
- VMD: Visual Molecular Dynamics, molecular graphics software
- YAP: Yes-associated protein

## Claims

1. A compound of formula 1: 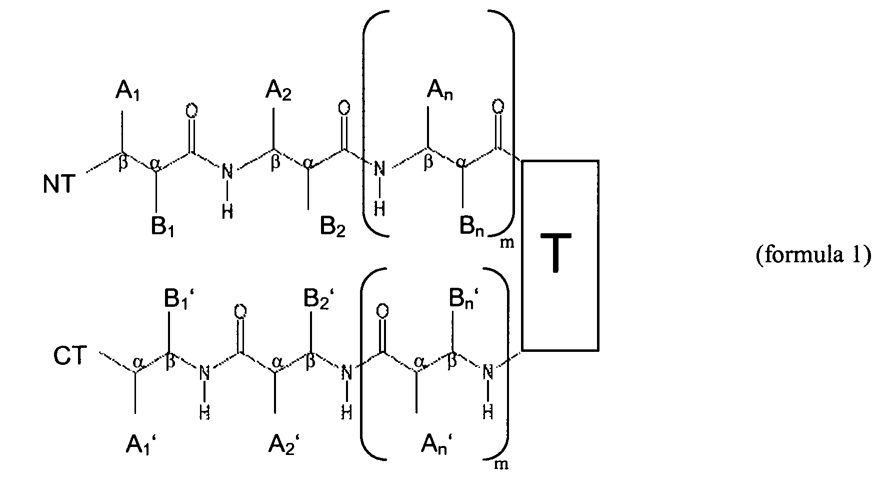 comprising an N-terminal oligomer and a C-terminal oligomer, which are connected covalently to a turn motive T wherein each oligomer consist of at least three β-amino acid residues as monomers,
m is an integer from 1 to 6, n and n' are the indexes of the third and if m > 1 subsequent monomers with integers from 3 to 8,
NT is the N-terminus of monomer 1, which is either a free or a modified amino group,
CT is the C terminus of monomer 1', which is either a free or a modified carboxyl group
all or all but m - 1 monomers in the C-terminal oligomer are heterochiral β^{2,3}-amino acid residues and all or all but m - 1 monomers in the N-terminal oligomer are heterochiral β^{2,3}-amino acid residues,
all or all but m - 1 side chains A₁, A₂ and Aₙ, B₁, B₂, Bₙ have at least 2 non-hydrogen atoms, and all or all but m - 1 side chains A₁', A₂' and A_{n'}, B₁', B₂' and Bₙ' have at least 2 non-hydrogen atoms,
at least four of the side chains chosen either from A₁ and A₁^{'}, A₂ and A₂^{'}, Aₙ and Aₙ^{'} or from B₁ and B₁^{'}, B₂ and B₂^{'}, Bₙ and Bₙ^{'}, form two pairs, wherein each non-prime member of the pair interacts with its prime (') counterpart, whereas at least one pair forms a polar interaction or a chemical bond and at least a second pair interacts via nonpolar forces.

2. A compound according to claim 1 forming a β-hairpin like structure with two distinct sides, wherein the side chains A₁, A₂ and Aₙ as well as the side chains A₁^{'}, A₂^{'} and Aₙ^{'} point to the one side of the β-hairpin and the side chains B₁, B₂ and Bₙ as well as the side chains B₁^{'}, B₂^{'} and Bₙ^{'} point to the opposite side of the β-hairpin.

3. A compound according to claim 1 or 2, wherein the polar interaction is a hydrogen bond, salt-bridge or Cation-π-interaction and the nonpolar interaction is an interaction based on van der Waals forces or an π-π-interaction.

4. A compound according to one of the claims 1 to 3 containing at least 2 hydrogen bonds which are formed between:
• N-H in the N-terminus NT with O in the C-terminus or N-H in the backbone of monomer 1' with O in the backbone of monomer 1,
• N-H in the backbone of residue 2 with O in the backbone of monomer 2' or N-H in the backbone of monomer 2' with O in the backbone of monomer 2,
• N-H in the backbone of residue n with O in the backbone of monomer n' or N-H in the backbone of monomer n' with O in the backbone of monomer n.

5. A compound according to one of the claims 1 to 4 wherein NT has the general formula NR₁R₂ with R₁ and R₂ independent from each other selected from hydrogen or the groups comprising:
a.) a beta-aminoacid building block or a chain of two or three beta-aminoacids;
b.) a straight chain, branched, cyclic or heterocyclic alkyl or alkanoyl group,
c.) a reporter group or a fusion tag or
d.) a bridge to CT to obtain a cyclic peptide.

6. A compound according to one of the claims 1 to 5 wherein CT has the general formula COR₃ or CSR₃ with R₃ selected from the groups comprising:
a.) a beta-aminoacid building block or a chain of two or three beta-aminoacids;
b.) hydroxyl, alkoxy, alkyl, sulfanyl and amine;
c.) a reporter group or a fusion tag or
d.) a bridge to NT to obtain a cyclic peptide.

7. A compound according to one of the claims 1 to 5 and according to the general formula 1'
NT-BA₁B₁-BA₂B₂-(BAₙBₙ)m-T-(BBₙ^{'}Aₙ^{'})ₘ-BB₂^{'}A₂^{'}-BB₁^{'}A₁^{'}-CT (formula 1')
wherein each β-amino acid building block is indicated by a three letter code with the first letter B indicating the β-amino acid, the second letter indicating the side chain of the C_{β} atom and the third letter indicating the side chain of the C_{α} atom, wherein the definitions of CT, NT, the side chains, the turn motive T and the indexes n and n' and m correspond to claim 1.

8. A compound according to claim 7 with m = 2 according to the general formula 20
NT-BA₁B₁-BA₂B₂-BA₃B₃-BA₄B₄-T-BB₄^{'}A₄^{'}-BB₃^{'}A₃^{'}-BB₂^{'}A₂^{'}-BB₁^{'}A₁^{'}-CT (formula 20)
wherein A₁, A₂, A₃ and A₄, as well as A₁', A₂', A₃' and A₄' are variable
B₁ is an nonpolar amino acid side chain,
B₂ is an aromatic amino acid side chain,
B₃ is a nonpolar amino acid side chain,
B₄ is a positively charged amino acid side chain,
B₄' is a negatively charged amino acid side chain or a polar side chain,
B₃' is a nonpolar amino acid side chain,
B₂' is a positively charged amino acid side chain,
B₁' is an nonpolar amino acid side chain.

9. A compound according to one of the claims 1 to 8, wherein the turn motive T comprises
(i) two mono-substituted β-amino acid residues forming a dipeptide sequence, preferably being a (S)-β²-(S)-β³-peptide or an (R)-β²-(R)-β³-peptide,
(ii) two amino acid residues, whereas the backbone is part of a 5, 6 or 7-membered ring, forming a dipeptide sequence, the amino acid residues being α-amino acid residues, β-amino acid residues or γ-amino acid residues, or
(iii) a ring system containing one, two or three 5 to 7 membered aliphatic or aromatic rings.

10. A compound according to claim 9 wherein the turn motive has the general formula 5 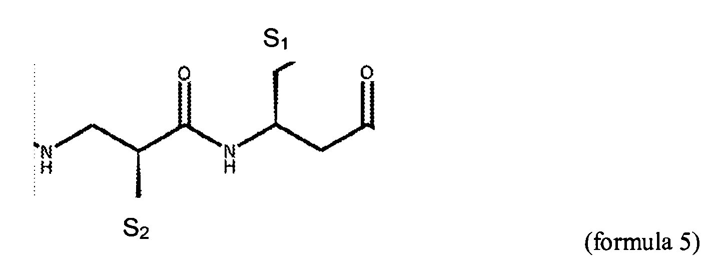 with S₁ and S₂ either in (R,R) or (S,S) configuration and being independently selected from natural or synthetic amino acid side chains, preferably one side chain being nonpolar and the other side chain polar, negatively or positively charged.

11. Use of a compound according to one of the claims 1 to 10 in pharmacy or biotechnology or as research tool in science, in particular to mimick a protein domain or a protein binding domain, to promote or inhibit the interaction of proteins and to modulate signaling pathways.

12. Pharmaceutical composition containing at least one compound according to one of the claims 1 to 10.

## Patentansprüche

1. Verbindung gemäß Formel 1 : 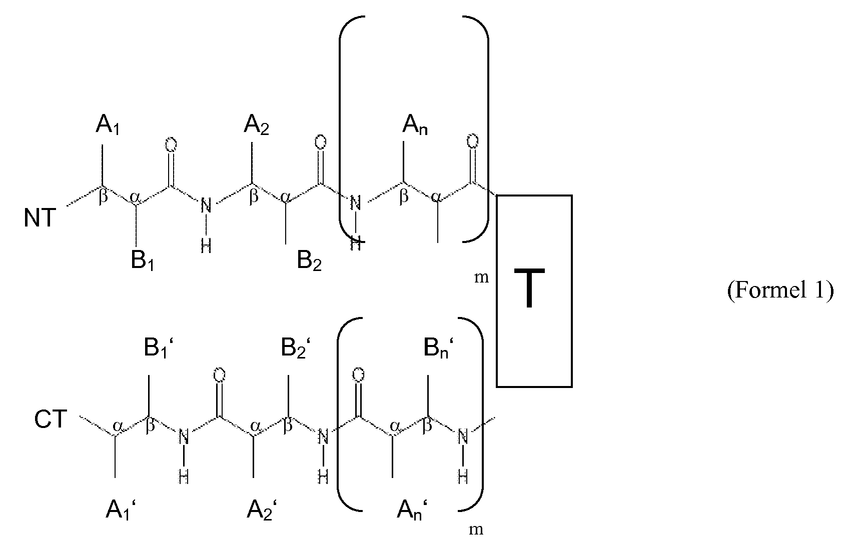 enthaltend ein N-terminales Oligomer und ein C-terminales Oligomer, welche kovalent mit einem Tummotiv T verbunden sind, wobei jedes Oligomer aus mindestens drei β-Aminosäureresten als Monomere besteht, wobei
m eine ganze Zahl von 1 bis 6 ist, n und n' die Indizes der dritten und wenn m > 1 der nachfolgenden Monomere sind, mit ganzen Zahlen von 3 bis 8,
NT der N-Terminus des Monomers 1 ist, welcher entweder eine freie oder modifizierte Aminogruppe ist,
CT der C-Terminus des Monomers 1' ist, welcher entweder eine freie oder modifizierte Carboxylgruppe ist,
alle oder alle bis auf m - 1 Monomere in dem C-terminalen Oligomer heterochirale β^{2,3} - Aminosäurereste sind und alle oder alle bis auf m - 1 Monomere in dem N-terminalen Oligomer heterochirale β^{2,3}- Aminosäurereste sind,
alle oder alle bis auf m - 1 Seitenketten A₁, A₂ und Aₙ, B₁, B₂, Bₙ mindestens 2 Nicht-Wasserstoff-Atome aufweisen, und alle oder alle bis auf m - 1 Seitenketten A₁ A₂, und Aₙ', B₁', B₂' und Bₙ' mindestens 2 Nicht-Wasserstoff-Atome aufweisen,
mindestens 4 der Seitenketten ausgewählt entweder aus A₁ und A₁^{'}, A₂ und A₂^{'}, Aₙ und Aₙ^{'} oder aus B₁ und B₁^{'}, B₂ und B₂^{'}, Bₙ und Bₙ^{'} zwei Paare bilden, in denen jeder Nicht-Strich-Partner des Paares mit seinem Strich-(')-Gegenpart wechselwirkt, wobei mindestens ein Paar eine polare Wechselwirkung oder eine chemische Bindung eingeht und mindestens ein zweites Paar durch unpolare Kräfte wechselwirkt.

2. Verbindung gemäß Anspruch 1, welche eine β-Haarnadelschleifen-ähnliche Struktur mit zwei unterschiedlichen Seiten bildet, wobei die Seitenketten A₁, A₂ und Aₙ sowie die Seitenketten A₁^{'}, A₂^{'} und Aₙ^{'} auf eine Seite der β-Haarnadelschleife und die die Seitenketten B₁, B₂ und Bₙ sowie die Seitenketten B₁^{'}, B₂^{'} und Bₙ^{'} zur gegenüberliegenden Seite der β-Haarnadelschleife zeigen.

3. Verbindung gemäß Anspruch 1 oder 2, wobei die polare Wechselwirkung eine Wasserstoffbrücke, Salzbrücke oder Kation-π-Wechselwirkung und wobei die unpolare Wechselwirkung eine Wechselwirkung ist, die auf van der Waals-Kräften basiert, oder eine π-π-Wechselwirkung.

4. Verbindung gemäß einem der Ansprüche 1 bis 3, enthaltend mindestens 2 Wasserstoffbrücken, die gebildet werden zwischen:
• N-H im N-Terminus NT mit O im C-Terminus oder N-H im Rückgrat des Monomers 1' mit O im Rückgrat des Monomers 1,
• N-H im Rückgrat des Restes 2 mit O im Rückgrat des Monomers 2' oder N-H im Rückgrat des Monomers 2' mit O im Rückgrat des Monomers 2,
• N-H im Rückgrat des Restes n mit O im Rückgrat des Monomers n' oder N-H im Rückgrat des Monomers n' mit O im Rückgrat des Monomers n.

5. Verbindung gemäß einem der Ansprüche 1 bis 4, wobei NT die allgemeine Formel NR₁R₂ aufweist, mit R₁ und R₂ unabhängig voneinander ausgewählt aus Wasserstoff oder den Gruppen enthaltend:
a.) einen beta-Aminosäurebaustein oder eine Kette von zwei oder drei beta-Aminosäuren;
b.) eine geradkettige, verzweigte, zyklische oder heterozyklische Alkyl- oder Alkanoyl-Gruppe,
c.) eine Reportergruppe oder ein Fusionstag oder
d.) eine Brücke zu CT, so dass ein zyklisches Peptid erhalten wird

6. Verbindung gemäß einem der Ansprüche 1 bis 5, wobei CT die allgemeine Formel COR₃ oder CSR₃ aufweist, mit R₃ ausgewählt aus den Gruppen enthaltend:
a.) einen beta-Aminosäurebaustein oder eine Kette von zwei oder drei beta-Aminosäuren;
b.) Hydroxyl, Alkoxy, Alkyl, Sulfanyl und Amin;
c.) eine Reportergruppe oder ein Fusionstag oder
d.) eine Brücke zu NT, so dass ein zyklisches Peptid erhalten wird.

7. Verbindung gemäß einem der Ansprüche 1 bis 5 und gemäß der allgemeinen Formel 1'
NT-BA₁B₁-BA₁B₁-(BAₙBₙ)ₘ-T-(BBₙ^{'}Aₙ^{'})ₘ-BB₂^{'}A₂^{'}-BB₁^{'}A₁^{'}-CT (Formel 1')
wobei jeder β-Aminosäurebaustein durch einen Dreibuchstabencode dargestellt ist, wobei der erste Buchstabe B die β-Aminosäure angibt, der zweite Buchstabe die Seitenkette am C_{β}-Atom angibt und der dritte Buchstabe die Seitenkette am C_{α}-Atom angibt, wobei die Definitionen von CT, NT, den Seitenketten, des Tummotivs T und der Indizes n und n' und m denen in Anspruch 1 entsprechen.

8. Verbindung gemäß Anspruch 7 mit m = 2 gemäß der allgemeinen Formel 20
NT-BA₁B₁-BA₂B₂-BA₃B₃-BA₄B₄-T-BB₄^{'}A₄^{'}-BB₃^{'}A₃^{'}-BB₂^{'}A₂^{'}-BB₁^{'}A₁^{'}-CT (Formel 20)
wobei A₁, A₂, A₃ und A₄, sowie A₁', A₂', A₃' und A₄' variabel sind,
B1 eine unpolare Aminosäureseitenkette ist,
B₂ eine aromatische Aminosäureseitenkette ist,
B₃ eine unpolare Aminosäureseitenkette ist,
B₄ eine positiv geladene Aminosäureseitenkette ist,
B₄' eine negative geladene Aminosäureseitenkette oder eine polare Seitenkette ist,
B₃' eine unpo lare Amino säureseitenkette ist,
B₂' eine positiv geladene Aminosäureseitenkette ist,
B₁' eine unpolare Aminosäureseitenkette ist.

9. Verbindung gemäß einem der Ansprüche 1 bis 8, wobei das Tummotiv T
(i) zwei mono-substituierte β-Aminosäurereste, welche eine Dipeptidsequenz bilden, bevorzugt ein (S)-β²-(S)-β³-Peptid oder ein (R)-β²-(R)-β³-Peptid,
(ii) zwei Aminosäurereste, in welchen das Rückgrat Teil eines 5-, 6- oder 7-gliedrigen Ringes ist, welche eine Dipeptidsequenz bilden, wobei die Aminosäurereste α- Aminosäurereste, β- Aminosäurereste oder γ-Aminosäurereste sind, oder
(iii) ein Ringsystem enthaltend einen, zwei oder drei 5- bis 7-gliedrige aliphatische oder aromatische Ringe
enthält.

10. Verbindung gemäß Anspruch 9, in der das Tummotiv die allgemeine Formel 5 aufweist 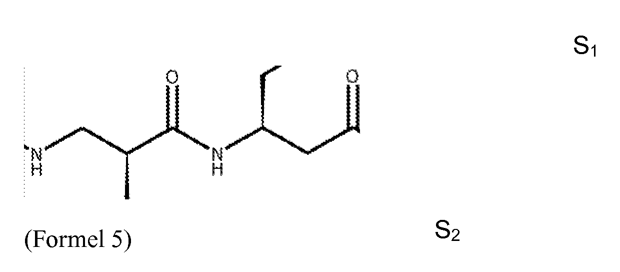 wobei S₁ und S₂ entweder (R,R)- oder (S,S)-Konfiguration aufweisen und unabhängig voneinander ausgewählt sind aus natürlichen oder synthetischen Aminosäurenseitenketten, wobei vorzugsweise eine Seitenkette unpolar und die andere Seitenkette polar, negativ oder positiv geladen ist.

11. Verwendung einer Verbindung gemäß einem der Ansprüche 1 bis 10 in der Pharmazie oder Biotechnologie oder Hilfsmittel für die wissenschaftliche Forschung, insbesondere um eine Proteindomäne oder eine Proteinbindungsdomäne nachzubilden, um die Wechselwirkung zwischen Proteinen zu fördern oder zu hemmen und um Signalübertragungswege zu modulieren.

12. Pharmazeutische Zusammensetzung enthaltend mindestens eine Verbindung nach einem der Ansprüche 1 bis 11.

## Revendications

1. Composé de formule 1 : 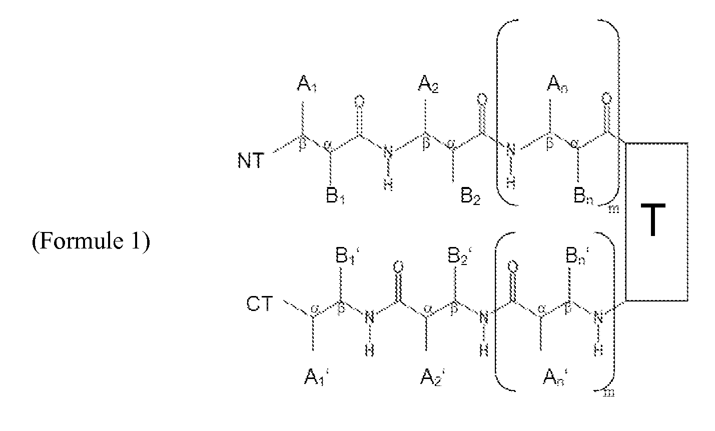 comprenant un oligomère N-terminal et un oligomère C-terminal qui sont reliés par une liaison covalente à un motif de pivotement T, où chaque oligomère est constitué d'au moins trois résidus de β-acides aminés en tant que monomères,
m est un entier allant de 1 à 6, n et n' sont les indices du troisième monomère et si m > 1 des monomères ultérieurs avec des entiers allant de 3 à 8,
NT est la N-terminaison du monomère 1, qui est soit un groupement amino libre, soit un groupement amino modifié,
CT est la C-terminaison du monomère 1', qui est soit un groupement carboxyle libre, soit un groupement carboxyle modifié,
tous les monomères ou tous les monomères sauf m-1 dans l'oligomère C-terminal sont des résidus de β^{2,3}-acides aminés hétérochiraux et tous les monomères ou tous les monomères sauf m-1 dans l'oligomère N-terminal sont des résidus de β^{2,3}-acides aminés hétérochiraux,
toutes les chaînes latérales A₁, A₂ et Aₙ, B₁, B₂, Bₙ ou toutes les chaînes latérales A₁, A₂ et Aₙ, B₁, B₂, Bₙ sauf m-1 ont au moins 2 atomes autres qu'un hydrogène, et toutes les chaînes latérales ou toutes les chaînes latérales sauf-1, A'₁, A'₂ et A'ₙ, B'₁ B'₂ et B'ₙ ont au moins 2 atomes autres qu'un hydrogène,
au moins quatre des chaînes latérales choisies soit à partir de A₁ et A' 1, A₂ et A'₂, Aₙ et A'ₙ soit à partir de B₁ et B'₁, B₂ et B'₂, Bₙ et B'ₙ forment deux paires, où chaque élément sans prime de la paire interagit avec son homologue avec un prime ('), où au moins une première paire forme une interaction polaire ou une liaison chimique et au moins une deuxième paire interagit par l'intermédiaire de forces non polaires.

2. Composé selon la revendication 1, formant une structure du type β-hairpin (ou épingle à cheveux) avec deux chaînes latérales distinctes, où les chaînes latérales A₁, A₂ et Aₙ de même que les chaînes latérales A'₁, A'₂ et A'ₙ sont orientées ou pointent en direction d'un premier côté du β-hairpin et les chaînes latérales B₁, B₂ et Bₙ de même que les chaînes latérales B'₁, B'₂, et B'ₙ sont orientées en direction du côté opposé du β-hairpin.

3. Composé selon la revendication 1 ou 2, dans lequel l'interaction polaire est une liaison hydrogène, un pont de sel ou une interaction cation-π et l'interaction non-polaire est une interaction basée sur les forces de Van der Waals ou sur une interaction π-π.

4. Composé selon l'une des revendications 1 à 3, contenant au moins 2 liaisons hydrogène qui sont formées entre :
- N-H dans la N-terminaison NT avec O dans la C- terminaison ou N-H dans le squelette du monomère l'avec O dans le squelette du monomère 1,
- N-H dans le squelette du résidu 2 avec O dans le squelette du monomère 2' ou N-H dans le squelette du monomère 2' avec O dans le squelette du monomère 2,
- N-H dans le squelette du résidu n avec O dans le squelette du monomère n' ou N-H dans le squelette du monomère n' avec O dans le squelette du monomère n.

5. Composé selon l'une des revendications 1 à 4 dans lequel NT a la formule générale NR₁R₂, R₁ et R₂ étant indépendants l'un de l'autre, sélectionnés à partir d'un hydrogène ou des groupements comprenant :
a.) un bloc de construction de bêta-acides aminés ou une chaîne de deux ou trois bêta-acides aminés,
b.) un groupement alkyle ou alcanoyle à chaîne linéaire, ramifié, cyclique ou hétérocyclique,
c.) un groupement rapporteur ou une balise de fusion ou
d.) un pont vers CT pour obtenir un peptide cyclique.

6. Composé selon l'une des revendications 1 à 5, dans lequel CT présente la formule générale COR₃ ou CSR₃, R₃ étant sélectionné à partir des groupes comprenant:
a.) un bloc de construction de bêta-acides aminés ou une chaîne de deux ou trois bêta-acides aminés,
b.) un hydroxyle, un alkoxy, un alkyle, un sulfanyle, et une amine,
c.) un groupement rapporteur ou une balise de fusion ou
d.) un pont vers NT pour obtenir un peptide cyclique.

7. Composé selon l'une des revendications 1 à 5 et conformément à la formule générale 1'
NT-BA₁B₁-BA₂B₂-(BAₙBₙ)ₘ-T-(BB'ₙAₙ)ₘ-BB'₂A'₂-BB'₁A'₁-CT (formule 1')
où chaque bloc de construction de β-acides aminés est indiqué par un code à trois lettres, la première lettre B indiquant le β-acide aminé, la deuxième lettre indiquant la chaîne latérale de l'atome C_{β} et la troisième lettre indiquant la chaîne latérale de l'atome C_{α}, où définitions de CT, NT, des chaînes latérales, du motif de pivotement T et des indices n et n' et m les correspondent à la revendication 1.

8. Composé selon la revendication 7, avec m = 2 conformément à la formule générale 20
NT-BA₁B₁-BA₂B₂-BA₃B₃-BA₄B₄-T-BB'₄A'₄-BB'₃A'₃-BB'₂A'₂-BB'₁A'₁-CT (formule 20)
où A₁, A₂, A₃ et A₄, de même que A'₁, A'₂, A'₃ et A'₄ sont variables
B₁ est une chaîne latérale d'acides aminés non polaire,
B₂ est une chaîne latérale d'acides aminés aromatique,
B₃ est une chaîne latérale d'acides aminés non polaire,
B₄ est une chaîne latérale d'acides aminés chargée positivement,
B'₄ est une chaîne latérale d'acides aminés chargée négativement ou une chaîne latérale polaire,
B'₃ est une chaîne latérale d'acides aminés non polaire,
B'₂ est une chaîne latérale d'acides aminés chargée positivement,
B'₁ est une chaîne latérale d'acides aminés non polaire.

9. Composé selon l'une des revendications 1 à 8, dans lequel le motif de pivotement T comprend :
(i) deux résidus β-acides aminés mono-substitués formant une séquence dipeptidique, de préférence un peptide (S)-β²-(S)-β³ ou un peptide (R)-β²-(R)-β³,
(ii) deux résidus d'acides aminés, où le squelette fait partie d'un cycle à 5, 6 ou 7 atomes, formant une séquence dipeptidique, les résidus d'acides aminés étant des résidus d'α-acides aminés, des résidus de β-acides aminés ou des résidus de γ-acides aminés ou,
(iii) un système de cycle contenant un, deux ou trois cycles aliphatiques ou aromatiques à 5 à 7 atomes.

10. Composé selon la revendication 9, dans lequel le motif de pivotement présente la formule générale 5 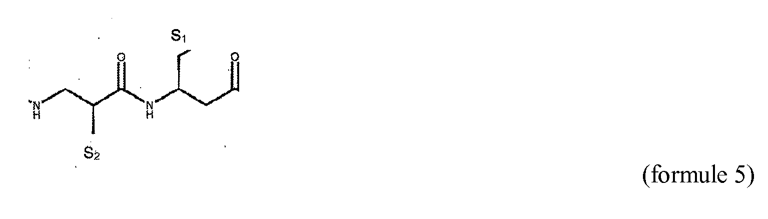 S₁ et S₂ étant soit dans la configuration (R,R) soit dans la configuration (S,S) et étant indépendamment sélectionnés à partir de chaînes latérales d'acides aminés naturels ou synthétiques, de préférence une première chaîne latérale étant non polaire et l'autre chaîne latérale étant polaire, chargée négativement ou positivement.

11. Utilisation d'un composé selon l'une des revendications 1 à 10 en pharmacie ou dans les biotechnologies ou à titre d'outil de recherche en science en particulier pour imiter un domaine de protéine ou un domaine de liaison de protéine, pour favoriser ou inhiber l'interaction de protéines et pour moduler les voies de signalisation.

12. Composition pharmaceutique contenant au moins un composé selon l'une des revendications 1 à 10.


### SEQUENCE LISTINGS

<110> Technische Universität Dresden
<120> Modular scaffold for the design of specific molecules for the use as peptidomimetics and inhibitors of protein interaction
<130> 00017P0107EP
<160> 3
<170> PatentIn version 3.5
<210> 1
   <211> 16
   <212> PRT
   <213> Artificial Sequence
<220>
   <223> mimetic of the YAP65 ww domain
<220>
   <221> misc_feature
   <222> (8)..(8)
   <223> Xaa is D-Proline
<220>
   <221> MISC_FEATURE
   <222> (16)..(16)
   <223> AMIDATION
<300>
   <301> Espinosa, J. F. et al.
   <303> Biopolymers
   <304> 80
   <306> 303-311
   <307> 2005
<400> 1
   Arg Tyr Phe Leu Asn His Val Xaa Gly Lys Gln Thr Thr Thr Trp Gln
   1 5 10 15
<210> 2
   <211> 12
   <212> PRT
   <213> Artificial Sequence
<220>
   <223> Peptide
<220>
   <221> MISC_FEATURE
   <222> (12)..(12)
   <223> AMIDATION
<400> 2
   Ser Trp Thr Trp Glu Asn Gly Lys Trp Thr Trp Lys
   1 5 10
<210> 3
   <211> 10
   <212> PRT
   <213> Artificial sequence
<220>
   <223> Peptide
<400> 3
   Gly Thr Pro Pro Pro Pro Tyr Thr Val Gly
   1 5 10